# EUROPEAN PATENT SPECIFICATION

(11) **EP 2 363 159 B1**
(45) Date of publication and mention of the grant of the patent: **24.10.2012**
(21) Application number: 11168666.3
(22) Date of filing: 17.08.2009
(51) Int. Cl.: A61M 5/145

(54) **Power injector with tilt sensor and syringe communication logic**
Hochdruckinjektor mit Neigungssensor und Schaltung zum Datenaustausch mit der Spritze
Injecteur haute pression avec capteur d'inclination et circuit de communication avec une seringue

(30) Priority: 19.08.2008 US 90020 P
(43) Date of publication of application: 07.09.2011
(62) Divisional of application: 09791556.5
(73) Proprietor: Mallinckrodt LLC, Hazelwood, MO 63042 (US)
(72) Inventor: Bruce, John K, Burlington, KY Kentucky 41005 (US); Gibson, Chad M, Westerville, OH Ohio 43081 (US); Strobl, Geoffrey S, Williamsburg, OH Ohio 45176 (US)
(74) Representative: Chettle, Adrian John

(56) References cited:
- WO-A-2006/108026
- WO-A-2006/109779
- US-A1- 2005 182 323

## Description

### FIELD OF THE INVENTION

The present invention generally relates to the field of power injectors and, more particularly, to controlling communications between a power injector and at least one power injector syringe.

### BACKGROUND

Various medical procedures require that one or more medical fluids be injected into the patient. Medical imaging procedures oftentimes involve the injection of a contrast media into the patient, possibly along with saline or other fluids. Other medical procedures involve injecting one or more fluids into a patient for therapeutic purposes. Power injectors may be used for these types of applications.

A power injector generally includes what is commonly referred to as a powerhead. One or more syringes may be mounted to the powerhead in various manners (e.g., detachably; rear-loading; front-loading; side-loading). Each syringe typically includes what may be characterized as a syringe plunger, piston, or the like. Each such syringe plunger is designed to interact with (e.g., contact and/or temporarily interconnect with) an appropriate syringe plunger driver that is incorporated into the powerhead, such that operation of the syringe plunger driver axially advances the associated syringe plunger inside and relative to a barrel of the syringe. One typical syringe plunger driver is in the form of a ram that is mounted on a threaded lead or drive screw. Rotation of the drive screw in one rotational direction advances the associated ram in one axial direction, while rotation of the drive screw in the opposite rotational direction advances the associated ram in the opposite axial direction.

RFID tags are becoming more and more popular in various applications. RFID tags have been addressed in relation to medical applications, and including in relation to power injectors. For instance, it has at least been suggested to dispose an RFID tag on a power injector syringe and encode at least certain information onto such an RFID tag. An RFID reader antenna on or associated with the power injector may be used to read the information from this syringe-mounted RFID tag.

WO-A-2006/109779 and US 2005182323 A1 both disclose an auto injector havinig an orientation sensor for indicating the inclination of a syringe on the auto-injector.

### SUMMARY

The present invention is embodied by a power injector according to claim 1 that includes a syringe plunger driver that in turn includes a motorized drive source, an orientation sensor, and a syringe communication module or logic. The syringe communication logic is operatively interconnected with the orientation sensor and is configured to initiate a syringe communication in response to the syringe communication logic identifying an occurrence of a first condition utilizing an output of the orientation sensor.

Any appropriate sensor that is capable of providing orientation information on the power injector may be utilized, the orientation sensor may be disposed at any appropriate location (e.g., on a powerhead of the power injector), including individually and in combination with each other. Multiple orientation sensors may be utilized and disposed in any appropriate arrangement. One embodiment has the orientation sensor being in the form of an accelerometer. Another embodiment has the orientation sensor being in the form of a tilt sensor. In any case, an output of the orientation sensor is used by the syringe communication logic to identify a predetermined change in orientation of at least part of the power injector, such as a powerhead, a syringe installed on the powerhead, or both.

The syringe communication logic is configured to initiate a syringe communication in response to an identification of a first condition by the syringe communication logic. This first condition is a predetermined change in orientation of at least part of the power injector (e.g., a powerhead, a syringe installed on a powerhead, or both). The predetermined change in orientation may be based upon a magnitude of an orientation change, the time over which an orientation change occurs, the direction of an orientation change, or any combination thereof. In one embodiment, the first condition is in the form of a threshold acceleration magnitude, alone or in combination with a direction or vector of the acceleration. Exposing a powerhead of the power injector to at least a certain acceleration, for instance moving the powerhead into a "tilted up" position or a "tilted down" position each may generate a first condition that will trigger a syringe communication.

At least one syringe may be installed on a powerhead of the power injector. The powerhead may be moved into a position so that a discharge nozzle of a power injector syringe projects at least generally upwardly (e.g., a "tilted up" configuration for the powerhead, or so that a syringe discharge nozzle is disposed "above horizontal"). In one embodiment, the powerhead is movable at least generally about a first axis to dispose a syringe in this "tilted up" configuration. In any case, fluid may be loaded into one or more syringes with the powerhead in this "tilted up" configuration, air may be purged from one or more syringes, interconnected tubing, or both with the powerhead in this "tilted up" configuration, or both. Each of these types of actions would typically be initiated in the preliminary stages of a medical imaging procedure, and in any case well prior to operation of any imaging equipment (e.g., an MR scanner) that could adversely interfere with syringe communications involving the power injector. Therefore, using a predetermined change in orientation of a power injector as a basis for initiating a syringe communication should reduce the potential of this syringe communication being adversely affected by (or adversely affecting) operation of imaging equipment, where the power injector and imaging equipment are being used for a medical imaging procedure.

A powerhead of the power injector may be moved into a position so that a discharge nozzle of a syringe installed on the powerhead projects at least generally downwardly (e.g., a "tilted down" configuration for the powerhead, or so that a syringe discharge nozzle is disposed "below horizontal"). In one embodiment, the powerhead is movable at least generally about a first axis to dispose a syringe in this "tilted down" configuration. In any case, the powerhead may be moved into this "tilted down" position in preparation for starting an injection as part of a medical imaging procedure. There should be sufficient amount of time between when the powerhead reaches a "tilted down" position and prior to initiating an injection through operation of the power injector for conducting a medical imaging procedure (where the power injector would also typically be operated prior to operating imaging equipment in an image acquisition mode) to allow for at least one syringe communication involving the power injector. Therefore, using this type of predetermined change in orientation of a power injector as a basis for initiating a syringe communication should reduce the potential of this syringe communication being adversely affected by (or adversely affecting) operation of imaging equipment, where the power injector and imaging equipment are being used for a medical imaging procedure.

A second aspect presented herein is embodied by a power injector that includes a syringe plunger driver that in turn includes a motorized drive source, an imaging energy output sensor, and a syringe communication module or logic. The imaging energy output sensor is configured to acquire data on operation of imaging equipment from an environment in which the power injector is located. The syringe communication logic is operatively interconnected with the imaging energy output sensor and is configured to initiate a syringe communication in response to the syringe communication logic identifying an occurrence of a first condition utilizing an output from the imaging energy output sensor.

A number of feature refinements and additional features are applicable to the second aspect. The following discussion is applicable to the second aspect, up to the start of the discussion regarding feature refinements and additional features that are applicable to each of the first and second aspects.

Consider the case of an imaging system that utilizes the power injector and imaging equipment, which in turn utilizes at least one imaging energy source (e.g., an RF transmission system of an MR scanner). The first condition may be when the imaging energy source is in an inactive state. Another option is for the first condition to be when the imaging energy source has been in an inactive state for at least a certain amount of time. The syringe communication logic may be further configured to first identify a pattern by which an energy imaging source is being cycled between active and inactive states, and to then predict a time that the energy imaging source should be in an active state in accordance with this pattern for purposes of triggering a syringe communication. In one example, the imaging energy output sensor is in the form of an RF read/write device that is also used by the syringe communication logic for syringe communications (e.g., to communicate with one or more syringe data tags of a syringe installed on the power injector).

The imaging energy output sensor may be of any appropriate size, shape, configuration, and/or type. In one embodiment, the imaging energy output sensor is in the form of an RFID read/write device (e.g., an RFID antenna). Such an RFID read/write device may be used to monitor the operation of imaging equipment, may be used for syringe communications (e.g., to send communications to and/or to receive communications from a syringe), or both.

A number of feature refinements and additional features are separately applicable to each of the above-noted first and second aspects. The following discussion is separately applicable to each of the first and second aspects, up to the start of the discussion of a third aspect.

The power injector may include a communication device of any appropriate size, shape, configuration, and/or type for providing a syringe communication functionality (e.g., via being operatively interconnected with the syringe communication logic). Such a communication device may be used to send communications to a syringe (e.g., to a syringe data tag), to receive communications from a syringe, or both. In one example, this communication device is in the form of an RFID read/write device that is operatively interconnected with the syringe communication logic. This RFID read/write device may be of any appropriate size, shape, configuration, and/or type, such as in the form of an RFID antenna. In one example, the power injector includes a powerhead, which in turn includes the syringe plunger driver and the noted RFID read/write device. At least one syringe may be installed on the power injector in any appropriate manner, at least one syringe may include at least one data tag of any appropriate size, shape, configuration, and/or type (e.g., an RFID tag), and the RFID read/write device may be configured to communicate with at least one syringe data tag of one or more syringes installed on the power injector.

The power injector may include a prefilled syringe sensor that is operatively interconnected with the syringe communication logic. The syringe communication logic may be further configured to initiate a syringe communication only in response to the case where the syringe communication logic both identifies an occurrence of the first condition (in accordance with either of the first and second aspects) and also identifies an occurrence of a second condition utilizing an output of the prefilled syringe sensor (i.e., both the first and second conditions must first be identified to trigger a syringe communication in this case). This second condition may be when there has been at least a certain amount of attenuation of a signal that has passed through a zone that would be occupied by a prefilled syringe when installed on the power injector. Hereafter, this will be referred to as a "prefilled syringe zone."

The prefilled syringe sensor may be of any appropriate size, shape, configuration, and/or type. The prefilled syringe sensor may include a transmitter, along with a receiver that is operatively interconnected with the syringe communication logic. The receiver may be positioned so that a signal from the transmitter will have to pass through the prefilled syringe zone to reach the receiver. Multiple receivers may be utilized by the prefilled syringe sensor, and furthermore may be disposed in any appropriate arrangement. The transmitter and each receiver may each be of any appropriate type, such as an RF antenna.

Installation of a prefilled syringe on the power injector may be used as a trigger for a syringe communication. One or more syringes are typically installed on a power injector in the preliminary stages of a medical imaging procedure, and in any case well prior to operation of any imaging equipment (e.g., an MR scanner) that could adversely interfere with syringe communications involving the power injector. Therefore, identifying when a prefilled syringe has been installed on a power injector and using this as a trigger for initiating a syringe communication should reduce the potential of this communication being adversely affected by (or adversely affecting) operation of imaging equipment, where the power injector and imaging equipment are being used for a medical imaging procedure.

The power injector may include a syringe clamp - a structure for holding, restraining, or otherwise securing a syringe in at least some manner on a powerhead. This syringe clamp may be of any appropriate size, shape, configuration, and/or type, but will typically include at least one clamp member that is movable to provide open and closed configurations for the syringe clamp. In one example, the syringe clamp is disposed about a least a substantial portion of a perimeter of a barrel of the corresponding syringe when the syringe clamp is in its closed configuration, although such may not be required in all instances. Generally, the syringe clamp may limit or restrain motion of the syringe within a plane that is orthogonal to a direction that a syringe plunger moves within the syringe barrel. Although the syringe clamp could exert a clamping force on the syringe barrel (e.g., an "inwardly" directed force - a force directed at least generally toward an axis along which the syringe plunger moves within the syringe barrel), the syringe clamp could be slightly spaced from the syringe barrel, in contact with the syringe barrel, or any combination thereof.

One or more syringe clamp sensors may be utilized to monitor a position or configuration of the above-noted syringe clamp, and at least one of these syringe clamp sensors may be operatively interconnected with the syringe communication logic. Each such syringe clamp sensor may be of any appropriate size, shape, configuration, and/or type. Representative syringe clamp sensors include a magnet/Hall effect sensor combination, an optical sensor, an electro-mechanical switch, a proximity sensor (e.g., inductive), and a potentiometer. The syringe communication logic may be configured to initiate a syringe communication only in response to the case where the syringe communication logic both identifies an occurrence of the first condition (e.g., in accordance with either of the first and second aspects) and also identifies an occurrence of a second condition using an output of a syringe clamp sensor (i.e., both the first and second conditions must first be identified to trigger a syringe communication in this case).

Identifying a syringe clamp being in a predetermined position or configuration may be used as a trigger for a syringe communication via the syringe communication logic. One or more syringes are typically installed on a power injector in the preliminary stages of a medical imaging procedure, and in any case well prior to beginning operation of any imaging equipment (e.g., an MR scanner) that could adversely interfere with syringe communications. This installation may involve moving a syringe clamp into a closed configuration to secure a syringe to the power injector. A number of other preparatory actions would typically be undertaken after the syringe(s) is/are installed on the power injector. Therefore, using a syringe clamp sensor to determine when a syringe clamp has been moved into a closed configuration (or at least moving towards a closed configuration) as a trigger for initiating a syringe communication should reduce the potential of the syringe communication being adversely affected by (or adversely affecting) operation of imaging equipment, where the power injector and imaging equipment are being used for a medical imaging procedure.

A third aspect presented is embodied by a power injector that includes a syringe plunger driver that in turn includes a motorized drive source, a transmitter, a receiver, and a prefilled syringe detection module or logic. The prefilled syringe detection logic is configured to assess a signal issued by the transmitter and thereafter received by the receiver for an occurrence of a first condition. The first condition in the case of the third aspect is at least a certain amount of attenuation of the signal when passing from the transmitter to the receiver. At least a certain amount of signal attenuation may be equated with a prefilled syringe having been installed on the power injector.

A number of feature refinements and additional features are applicable to the third aspect. The following discussion is applicable to the third aspect, up to the start of the discussion of a fourth aspect.

Each of the transmitter and receiver may be of any appropriate size, shape, configuration, and/or type. In one embodiment, the transmitter is in the form of an RF antenna. At least one of the transmitter and the receiver may be in the form of an RF antenna, thereby compassing each of the transmitter and receiver being in the form of an RF antenna. Multiple receivers may be utilized and disposed in any appropriate arrangement. In any case, at least one receiver may be positioned to receive the signal from the transmitter after passing through a prefilled syringe zone.

The prefilled syringe detection logic may assess the transmitted signal to determine whether there has been at least a certain amount of attenuation of this signal. The prefilled syringe detection logic may be operatively interconnected with at least the receiver for assessing signal attenuation. Although the transmitter could also be operatively interconnected with the prefilled syringe detection logic, one or more characteristics of the signal to be sent by the transmitter may be stored in memory or may be otherwise made available to the prefilled syringe detection logic for purposes of assessing signal attenuation. In one example, the prefilled syringe detection logic compares the strength of the signal as transmitted by the transmitter (e.g., via an operative interconnection between the transmitter and the prefilled syringe detection logic; by the prefilled syringe detection logic having a priori knowledge of the signal to be sent by the transmitter) with the strength of the signal as received by the receiver. Signal attenuation may be assessed in any appropriate manner.

The prefilled syringe detection logic may be used to provide any function or combination of functions. In one embodiment, the prefilled syringe detection logic is used to determine if a prefilled syringe has been installed on the power injector. Any identification of a prefilled syringe having been installed on the power injector by the prefilled syringe detection logic may be communicated to any appropriate personnel and in any appropriate manner. In one example, the prefilled syringe detection logic is used to trigger a syringe communication in response to the prefilled syringe detection logic identifying an occurrence of the first condition. The prefilled syringe detection logic could provide both of these functions for a power injector.

The power injector may include a syringe clamp that may be disposed in each of open and closed configurations, a syringe clamp sensor, and syringe clamp detection logic. This syringe clamp detection logic may be operatively interconnected with the syringe clamp sensor and may be configured to identify an occurrence of a second condition, where this second condition is when the syringe clamp is in a predetermined position or configuration.

The power injector may also include syringe communication logic. The syringe communication logic may be configured to initiate a syringe communication only in response to the case where both the prefilled syringe detection logic identifies an occurrence of the first condition and the syringe clamp detection logic identifies an occurrence of the second condition (i.e. both of the first and second conditions must first be identified to trigger a syringe communication in this case). The syringe communication logic may also be configured to initiate a syringe communication in response to either the prefilled syringe detection logic identifying an occurrence of the first condition or the syringe clamp detection logic identifying an occurrence of the second condition (i.e., only one of the first and second conditions needs to be identified to trigger a syringe communication in this case). The syringe communication logic may include either or both of the prefilled syringe detection logic and the syringe clamp detection logic.

As defined in claim 8, the invention comprises a method of operation for a power injector. A monitoring operation is initiated for purposes of identifying an occurrence of a first condition, where the first condition is the power injector experiencing a predetermined change in orientation. An RF syringe communication is initiated with a syringe data tag in response to an identification of an occurrence of the first condition.

A number of feature refinements and additional features are applicable to the present invention.

Any appropriate way of monitoring for an occurrence of the first condition may be utilized. One or more sensors may be utilized to provide data that may be monitored/analyzed to determine if there has been an occurrence of a first condition (e.g., an accelerometer, a tilt sensor, an orientation sensor). In one embodiment, a syringe communication is initiated when the power injector experiences an acceleration of at least a certain magnitude. In one embodiment, a syringe communication is initiated when the power injector experiences an acceleration of at least a certain magnitude, where this acceleration is in a certain direction (e.g., such that a powerhead of the power injector is moving toward a "tilted up" or "tilted down" position).

A syringe may be installed on the power injector. The power injector (e.g., a powerhead) thereafter may be moved such that a discharge nozzle of this syringe projects at least generally upwardly (e.g., being disposed "above horizontal"), and which may be referred to as a first orientation. A movement of the power injector into this first orientation may provide an occurrence of a first condition that will initiate a syringe communication. One or more operations may take place with the power injector in this first orientation. Fluid may be loaded into the syringe at this time, air may be purged from the syringe or interconnected tubing at this time, or both.

The power injector may be moved into a second orientation (e.g., from the first orientation), where a discharge nozzle of a syringe installed on the power injector projects at least generally downwardly (e.g., being disposed "below horizontal"). Fluid may be discharged from the syringe some time after the power injector has been moved into its second orientation. A movement of the power injector into the second orientation may provide an occurrence of a first condition that will initiate a syringe communication, and which may occur before the fluid discharge from the syringe is actually initiated (e.g., there may be sufficient time between when the powerhead is positioned in its "tilted down" configuration and when an injection is actually started via operation of the power injector).

A fifth aspect presented is embodied by a method of operation for a medical system that includes imaging equipment, a power injector, and a syringe that is installed on the power injector and that includes a syringe data tag. Energy may be output from the imaging equipment at one or more times to acquire a medical image. The environment exposed to this energy output from the imaging equipment is monitored for an occurrence of a first condition. A syringe communication with a syringe data tag is initiated in response to an identification of an occurrence of a first condition.

A number of feature refinements and additional features are applicable to the fifth aspect. The following discussion is applicable to the fifth aspect, up to the start of the discussion of feature refinements/additional features that are separately applicable to each of the fifth and sixth aspects.

The energy output that is monitored may be of any appropriate form, including without limitation RF signals emitted by an MR scanner or the like. The energy output that is monitored for an occurrence of a first condition may be obtained from an environment in which the power injector is located. In one embodiment, the power injector provides the monitoring function in relation to the energy output from the imaging equipment.

The first condition may be characterized as being when the energy output from the imaging equipment is at least substantially zero, when an imaging energy source of the imaging equipment is in an inactive state, when an imaging energy source of the imaging equipment has been in an inactive state for at least a certain amount of time, when the imaging equipment is in a mode other than an image acquisition mode, or any combination thereof. Another option is for the first condition to be based upon a pattern. In this regard, the environment may be monitored to first identify a pattern being used to cycle an imaging energy source of the imaging equipment between on/off or active/inactive states. Once this pattern has been identified, a syringe communication may be initiated at a time when an imaging energy source of the imaging equipment should be in an off or inactive state in accordance with the previously identified pattern.

The environment may be monitored in any appropriate manner in relation to the energy output from the imaging equipment. In one embodiment, the power injector includes an RFID read/write device (e.g., an RFID antenna) that provides this monitoring function, and that is also able to communicate with a syringe data tag when in the form of a syringe RFID tag. As such, the RFID read/write device may monitor the operation of the imaging equipment to acquire information used to trigger a syringe communication, and also may be used for such a syringe communication.

A number of feature refinements and additional features are applicable to each of the invention and fifth aspect. The following discussion is separately applicable to each of the invention and fifth aspect, up to the start of the discussion of a sixth aspect.

A signal may be sent from a first location, may be received at a second location, and may be monitored after its receipt for an occurrence of a second condition. This second condition may be in the form of minimum signal attenuation (e.g., a determination as to whether there been at least a certain amount of attenuation of the signal between the time of its transmission and the time of its receipt). Although this signal for purposes of a second condition assessment may be of any appropriate type, in one embodiment the signal is in the form of an RF signal.

The signal may be transmitted through a prefilled syringe zone. As such, the signal travels from the first location, through the prefilled syringe zone, and to the second location. In one embodiment, the second condition occurs when there has been at least a certain amount of attenuation of the signal after being transmitted from the first location and until being received at the second location. In one embodiment, a syringe communication is initiated with a syringe data tag only for the case where each of the first condition (e.g., in accordance with either of the invention and fifth aspect) and the second condition has been identified (i.e., each of the first and second conditions must first be identified before a syringe communication may be initiated in this case).

The power injector may include a syringe clamp, and this syringe clamp may be monitored in any appropriate manner for an occurrence of a third condition. This third condition may be when the syringe clamp is in a predetermined configuration (e.g., a closed configuration; an open configuration; an intermediate configuration between its open and closed configurations). In one example, a syringe communication is initiated only for the case where each of the first condition (e.g., in accordance with either of the fourth and fifth aspects) and the third condition has been identified (i.e., each of the first and third conditions must first be identified before a syringe communication may be initiated in this case).

A sixth aspect presented is embodied by a method of operation for a power injector. A signal may be sent from a first location, may be received at a second location, and may be monitored after its receipt for an occurrence of a first condition. This first condition is in the form of a minimum signal attenuation - that is, there is a determination as to whether there been at least a certain amount of attenuation of the signal from the time the signal was originally transmitted and the time this signal was received. This first condition is equated with a prefilled syringe having been installed on the power injector.

A number of feature refinements and additional features are applicable to the sixth aspect. The following discussion is applicable to the sixth aspect, up to the start of the discussion of feature refinements and additional features that are separately applicable to each of the first through the sixth aspects.

Any appropriate signal may be utilized for purposes of the sixth aspect, including without limitation an RF signal. The signal may be transmitted through what a prefilled syringe zone. Identification of an occurrence of a first condition may be used for any purpose or combination of purposes. In one embodiment, at least one notification is issued to indicate in any appropriate manner that a prefilled syringe has been installed on the power injector. Identification of an occurrence of a first condition may be used to initiate a syringe communication - a communication with one or more syringe data tags of a prefilled syringe. Both of these actions may be undertaken when a first condition has been identified.

The power injector may include a syringe clamp, and this syringe clamp may be monitored in any appropriate manner for an occurrence of a second condition. This second condition may be when the syringe clamp is in a predetermined position or configuration (e.g., a closed configuration; an open configuration; an intermediate configuration between its open and closed configurations). In one embodiment, a syringe communication (e.g., a communication with at least one syringe data tag) is initiated only for the case when each of the first condition (e.g., installation of a prefilled syringe) and the second condition (e.g., a syringe clamp being in a predetermined configuration) has been identified (i.e., each of the first and second conditions must first be identified to initiate a syringe communication in this case). In another example, a syringe communication (e.g., a communication with at least one syringe data tag) is initiated when either the first condition (e.g., installation of a prefilled syringe) or the second condition (e.g., a syringe clamp being in a predetermined configuration) has been identified (i.e., only one of the first and second conditions needs to be identified to initiate a syringe communication in this case).

A number of feature refinements and additional features are separately applicable to each of the above-noted first through the sixth aspects.

Any "logic" that may be utilized by any of the various aspects of the present invention may be implemented in any appropriate manner, including without limitation in any appropriate software, firmware, or hardware, using one or more platforms, using one or more processors, using memory of any appropriate type, using any single computer of any appropriate type or a multiple computers of any appropriate type and interconnected in any appropriate manner, or any combination thereof. This logic may be implemented at any single location or at multiple locations that are interconnected in any appropriate manner (e.g., via any type of network).

A "syringe communication" encompasses a communication sent to a syringe, a communication received from a syringe, or both. Syringe communications may be of any appropriate type and of any appropriate form but according to the invention it comprises an RF signal. Any appropriate communication device (e.g., an RFID read/write device, such as an RF antenna) may be implemented in any appropriate manner by the power injector to send a syringe communication to a syringe, to receive a syringe communication from a syringe, or both. Multiple communication devices may be used by the power injector and may be disposed in any appropriate arrangement.

One or more syringes used by a power injector may include any appropriate data storage device for communicating with a power injector (e.g., via a syringe communication). References herein to a "syringe data tag" are intended to cover any appropriate structure or combination of structures for storing information on a syringe, in any appropriate manner, and at any appropriate location or combination of locations on the syringe (e.g., an RFID tag). Any appropriate information may be stored on a syringe data tag and in any appropriate manner. Each syringe used by a power injector may have any appropriate number of syringe data tags. It should be appreciated that not every syringe installed on a power injector needs to have at least one syringe data tag, although such could be the case.

A syringe clamp being in a "predetermined position or configuration" may be a trigger condition for initiating a syringe communication. A syringe clamp may be moved into a stationary position that corresponds with such a predetermined position or configuration (e.g., an open or closed configuration). However, the syringe clamp could be moving at a time when the syringe clamp is in a predetermined position or configuration that is a trigger condition for initiating a syringe communication. A certain movement of the syringe clamp could in fact be a predetermined position or configuration that is a trigger condition for initiating a syringe communication. Therefore, it is not required that a syringe clamp be in a stationary state or configuration to satisfy a "trigger condition" for a syringe communication.

The power injector may be of any appropriate size, shape, configuration, and/or type. The power injector may utilize one or more syringe plunger drivers of any appropriate size, shape, configuration, and/or type, where each such syringe plunger driver may be capable of at least bi-directional movement (e.g., a movement in a first direction for discharging fluid; a movement in a second direction for accommodating a loading of fluid or so as to return to a position for a subsequent fluid discharge operation), and where each such syringe plunger driver may interact with its corresponding syringe plunger in any appropriate manner (e.g., by mechanical contact; by an appropriate coupling (mechanical or otherwise)) so as to be able to advance the syringe plunger in at least one direction (e.g., to discharge fluid). Each syringe plunger driver may utilize one or more drive sources of any appropriate size, shape, configuration, and/or type. Multiple drive source outputs may be combined in any appropriate manner to advance a single syringe plunger at a given time. One or more drive sources may be dedicated to a single syringe plunger driver, one or more drive sources may be associated with multiple syringe plunger drivers (e.g., incorporating a transmission of sorts to change the output from one syringe plunger to another syringe plunger), or a combination thereof. Representative drive source forms include a brushed or brushless electric motor, a hydraulic motor, a pneumatic motor, a piezoelectric motor, or a stepper motor.

The power injector may be used for any appropriate application where the delivery of one or more medical fluids is desired, including without limitation any appropriate medical application (e.g., computed tomography or CT imaging; magnetic resonance imaging or MRI; single photon emission computed tomography or SPECT imaging; positron emission tomography or PET imaging; X-ray imaging; angiographic imaging; optical imaging; ultrasound imaging). The power injector may be used in conjunction with any component or combination of components, such as an appropriate imaging system (e.g., a CT scanner). For instance, information could be conveyed between any such power injector and one or more other components (e.g., scan delay information, injection start signal, injection rate).

Any appropriate number of syringes may be utilized with the power injector and in any appropriate manner (e.g., detachably; front-loaded; rear-loaded; side-loaded), any appropriate medical fluid may be discharged from a given syringe of the power injector (e.g., contrast media, a radiopharmaceutical, saline, and any combination thereof), and any appropriate fluid may be discharged from a multiple syringe power injector configuration in any appropriate manner (e.g., sequentially, simultaneously), or any combination thereof. In one embodiment, fluid discharged from a syringe by operation of the power injector is directed into a conduit (e.g., medical tubing), where this conduit is fluidly interconnected with the syringe in any appropriate manner and directs fluid to a desired location (e.g., to a catheter that is inserted into a patient, for instance for injection). Multiple syringes may discharge into a common conduit (e.g., for provision to a single injection site), or one syringe may discharge into one conduit (e.g., for provision to one injection site), while another syringe may discharge into a different conduit (e.g., for provision to a different injection site). In one embodiment, each syringe includes a syringe barrel and a plunger that is disposed within and movable relative to the syringe barrel. This plunger may interface with a power injector syringe plunger driver such that the syringe plunger driver is able to advance the syringe plunger in at least one direction, and possibly in two different, opposite directions.

### BRIEF DESCRIPTION OF THE FIGURES

Figure 1A is a schematic of one embodiment of a power injector.
Figure 1B is a perspective view of an injector head of an injector, having a syringe attached to a forward area thereof.
Figure 2A is an exploded view of one exemplary embodiment of a syringe mount.
Figure 2B is a perspective view of the syringe mount of Figure 2A in an assembled condition.
Figure 3A is a cutaway view of the syringe mount of Figure 2B, particularly showing an actuator of the syringe mount.
Figure 3B is a cross-sectional view, taken along line 3B-3B of Figure 3A.
Figure 4A is a cutaway view of syringe mount of Figure 2B, particularly showing first and second movable members of the syringe mount in an open position.
Figure 4B is a cross-sectional view, taken along line 4B-4B of Figure 4A, and also shows a coupling mechanism of a syringe plunger positioned in proximity to a plunger coupling element of a drive ram.
Figure 5A is a cutaway view of the syringe mount of Figure 2B, particularly showing the first and second movable members in a closed position and engaging a syringe.
Figure 5B is a cross-sectional view, taken along line 5B-5B of Figure 5A, and also shows the coupling mechanism on the backside of the syringe plunger engaged with the plunger coupling element of the drive ram.
Figure 6 is a schematic of one embodiment of an imaging system that utilizes a power injector, where this power injector includes syringe communication logic.
Figure 7 is one embodiment of a power injector communications protocol that may be used by the power injector of Figure 6.
Figure 8 is one embodiment of a monitoring protocol that may be used by the power injector communications protocol of Figure 7, where initiating a read/write communication is based upon a change in the orientation of a powerhead of the power injector.
Figure 9 is one embodiment of a monitoring protocol that may be used by the power injector communications protocol of Figure 7, where initiating a read/write communication is based upon a syringe clamp moving into/through a predetermined state/configuration.
Figure 10 is one embodiment of a monitoring protocol that may be used by the power injector communications protocol of Figure 7, where initiating a read/write communication is based upon a prefilled syringe being detected on the power injector through an attenuation analysis.
Figure 11A is one embodiment of a monitoring protocol that may be used by the power injector communications protocol of Figure 7, where initiating a read/write communication is based upon a monitoring of an output of an imaging energy source.
Figure 11B is one embodiment of a monitoring protocol that may be used by the power injector communications protocol of Figure 7, where initiating a read/write communication is based identifying a pattern of an output of an imaging energy source.

### DETAILED DESCRIPTION

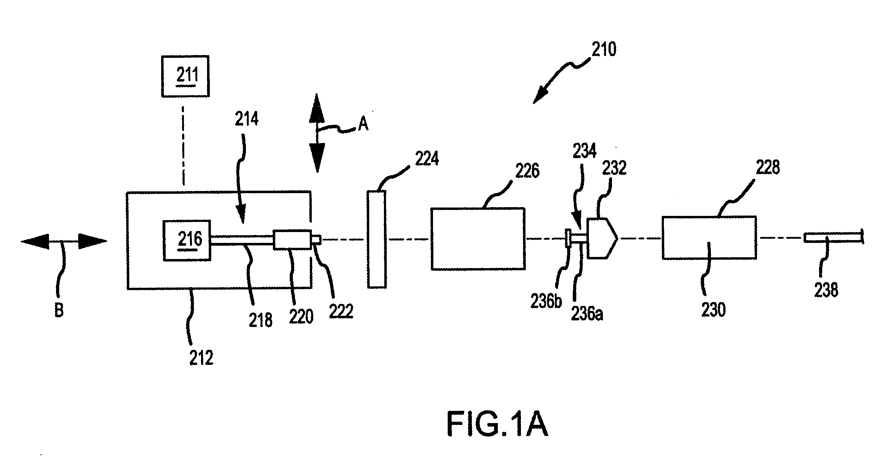

Figure 1A presents a schematic of one embodiment of a power injector 210 having a powerhead 212. One or more graphical user interfaces or GUIs 211 may be associated with the powerhead 212. Each GUI 211: 1) may be of any appropriate size, shape, configuration, and/or type; 2) may be operatively interconnected with the powerhead 212 in any appropriate manner; 3) may be disposed at any appropriate location; 4) may be configured to provide one or any combination of the following functions: controlling one or more aspects of the operation of the power injector 210; inputting/editing one or more parameters associated with the operation of the power injector 210; and displaying appropriate information (e.g., associated with the operation of the power injector 10); or 5) any combination of the foregoing. Any appropriate number of GUIs 211 may be utilized. In one embodiment, the power injector 210 includes a GUI 211 that is incorporated by a console that is separate from but which communicates with the powerhead 212. In another embodiment, the power injector 210 includes a GUI 211 that is part of the powerhead 212. In yet another embodiment, the power injector 210 utilizes one GUI 211 on a separate console that communicates with the powerhead 212, and also utilizes another GUI 211 that is on the powerhead 212. Each GUI 211 could provide the same functionality or set of functionalities, or the GUIs 211 may differ in at least some respect in relation to their respective functionalities.

A syringe 228 may be installed on this powerhead 212 and, when installed, may be considered to be part of the power injector 210. Some injection procedures may result in a relatively high pressure being generated within the syringe 228. In this regard, it may be desirable to dispose the syringe 228 within a pressure jacket 226. The pressure jacket 226 is typically associated with the powerhead 212 in a manner that allows the syringe 228 to be disposed therein as a part of or after installing the syringe 228 on the powerhead 212. The same pressure jacket 226 will typically remain associated with the powerhead 212, as various syringes 228 are positioned within and removed from the pressure jacket 226 for multiple injection procedures. The power injector 210 may eliminate the pressure jacket 226 if the power injector 210 is configured/utilized for low-pressure injections and/or if the syringe(s) 228 to be utilized with the power injector 210 is (are) of sufficient durability to withstand high-pressure injections without the additional support provided by a pressure jacket 226. In any case, fluid discharged from the syringe 228 may be directed into a conduit 238 of any appropriate size, shape, configuration, and/or type, which may be fluidly interconnected with the syringe 228 in any appropriate manner, and which may direct fluid to any appropriate location (e.g., to a patient).

The powerhead 212 includes a syringe plunger drive assembly or syringe plunger driver 214 that interacts (e.g., interfaces) with the syringe 228 (e.g., a plunger 232 thereof) to discharge fluid from the syringe 228. This syringe plunger drive assembly 214 includes a drive source 216 (e.g., a motor of any appropriate size, shape, configuration, and/or type, optional gearing, and the like) that powers a drive output 218 (e.g., a rotatable drive screw). A ram 220 may be advanced along an appropriate path (e.g., axial) by the drive output 218. The ram 220 may include a coupler 222 for interacting or interfacing with a corresponding portion of the syringe 228 in a manner that will be discussed below.

The syringe 228 includes a plunger or piston 232 that is movably disposed within a syringe barrel 230 (e.g., for axial reciprocation along an axis coinciding with the double-headed arrow B). The plunger 232 may include a coupler 234. This syringe plunger coupler 234 may interact or interface with the ram coupler 222 to allow the syringe plunger drive assembly 214 to retract the syringe plunger 232 within the syringe barrel 230. The syringe plunger coupler 234 may be in the form of a shaft 236a that extends from a body of the syringe plunger 232, together with a head or button 236b. However, the syringe plunger coupler 234 may be of any appropriate size, shape, configuration, and/or type.

Generally, the syringe plunger drive assembly 214 of the power injector 210 may interact with the syringe plunger 232 of the syringe 228 in any appropriate manner (e.g., by mechanical contact; by an appropriate coupling (mechanical or otherwise)) so as to be able to move or advance the syringe plunger 232 (relative to the syringe barrel 230) in at least one direction (e.g., to discharge fluid from the corresponding syringe 228). That is, although the syringe plunger drive assembly 214 may be capable of bi-directional motion (e.g., via operation of the same drive source 216), the power injector 210 may be configured such that the operation of the syringe plunger drive assembly 214 actually only moves each syringe plunger 232 being used by the power injector 210 in only one direction. However, the syringe plunger drive assembly 214 may be configured to interact with each syringe plunger 232 being used by the power injector 210 so as to be able to move each such syringe plunger 232 in each of two different directions (e.g. in different directions along a common axial path).

Retraction of the syringe plunger 232 may be utilized to accommodate a loading of fluid into the syringe barrel 230 for a subsequent injection or discharge, may be utilized to actually draw fluid into the syringe barrel 230 for a subsequent injection or discharge, or for any other appropriate purpose. Certain configurations may not require that the syringe plunger drive assembly 214 be able to retract the syringe plunger 232, in which case the ram coupler 222 and syringe plunger coupler 234 may not be desired. In this case, the syringe plunger drive assembly 214 may be retracted for purposes of executing another fluid delivery operation (e.g., after another pre-filled syringe 228 has been installed). Even when a ram coupler 222 and syringe plunger coupler 234 are utilized, it may such that these components may or may not be coupled when the ram 220 advances the syringe plunger 232 to discharge fluid from the syringe 228 (e.g., the ram 220 may simply "push on" the syringe plunger coupler 234 or on a proximal end of the syringe plunger 232). Any single motion or combination of motions in any appropriate dimension or combination of dimensions may be utilized to dispose the ram coupler 222 and syringe plunger coupler 234 in a coupled state or condition, to dispose the ram coupler 222 and syringe plunger coupler 234 in an un-coupled state or condition, or both.

The syringe 228 may be installed on the powerhead 212 in any appropriate manner. For instance, the syringe 228 could be configured to be installed directly on the powerhead 212. In the illustrated embodiment, a housing 224 is appropriately mounted on the powerhead 212 to provide an interface between the syringe 228 and the powerhead 212. This housing 224 may be in the form of an adapter to which one or more configurations of syringes 228 may be installed, and where at least one configuration for a syringe 228 could be installed directly on the powerhead 212 without using any such adapter. The housing 224 may also be in the form of a faceplate to which one or more configurations of syringes 228 may be installed. In this case, it may be such that a faceplate is required to install a syringe 228 on the powerhead 212 - the syringe 228 could not be installed on the powerhead 212 without the faceplate. When a pressure jacket 226 is being used, it may be installed on the powerhead 212 in the various manners discussed herein in relation to the syringe 228, and the syringe 228 will then thereafter be installed in the pressure jacket 226.

The housing 224 may be mounted on and remain in a fixed position relative to the powerhead 212 when installing a syringe 228. Another option is to movably interconnect the housing 224 and the powerhead 212 to accommodate installing a syringe 228. For instance, the housing 224 may move within a plane that contains the double-headed arrow A to provide one or more of coupled state or condition and an un-coupled state or condition between the ram coupler 222 and the syringe plunger coupler 234.

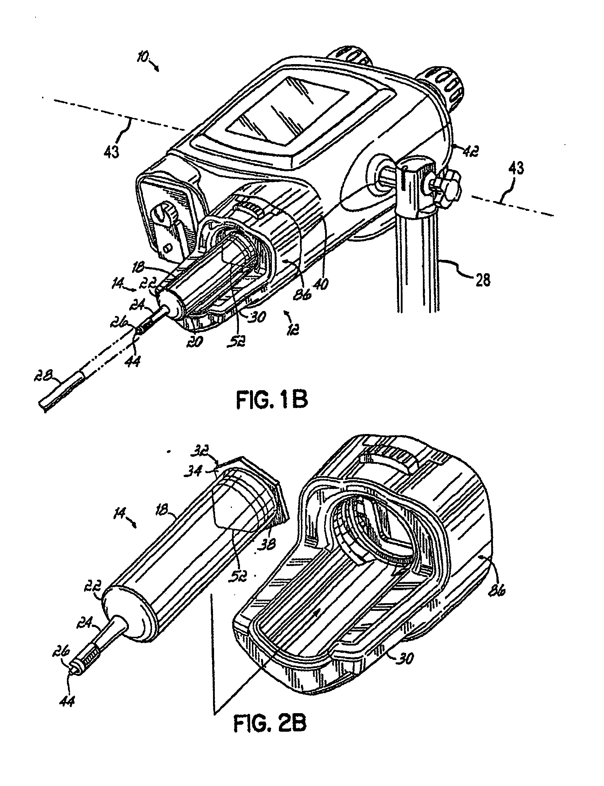

Referring to Figure 1B, a power injector 10 includes a housing or a powerhead 42 that may be mounted on a stand 28 (e.g., which may include a wheeled base or the like for transportability, not shown), on a wall or ceiling via an appropriate linkage or the like, or any other appropriate support. The powerhead 42 is pivotable about an axis 43, and may be pivoted and maintained in a desired orientation (e.g., via the illustrated knob) to provide any appropriate function. For instance and when a syringe 14 is installed on the powerhead 42, the powerhead 42 may be tilted into a position where a discharge tip 26 of the syringe 14 is above horizontal (e.g., such that the discharge tip 26 of the syringe 14 projects at least generally upwardly) to load fluid into the syringe 14, to purge air from the syringe 14 and/or any interconnected tubing, or both. The powerhead 42 may be tilted into a position where the discharge tip 26 of the syringe 14 is below horizontal (e.g., such that the discharge tip 26 of the syringe 14 projects at least generally downwardly) to discharge fluid from the syringe 14 (e.g., for injection into a patient via a catheter or the like).

The power injector 10 includes a syringe mount 12 to facilitate attachment of a syringe 14 to the injector 10 in alignment with a drive ram 16, in order to provide an injection assembly. The syringe 14 for use with the injector 10 generally includes a body 18 (which may be in the form of an exterior cylindrical barrel), which at its forward end 20, is integral with a conical front wall 22. A neck 24, terminating in a discharge tip 26, generally extends forwardly from and may be integral with the conical front wall 22. The body 18 of the syringe 14 may interface with an interior wall of a pressure jacket (not shown) or a cradle 30 when such a pressure jacket or cradle 30 is present on the injector 10. The syringe 14, as used in conjunction with the injector 10, includes a syringe mating section 32, which may be in the form of a radially outwardly extending flange 34. This flange 34 is positioned in a plane substantially perpendicular to a longitudinal axis 36 of the syringe 14 and may generally be integral with the rearward end 38 of the body 18 of the syringe 14. When the syringe 14 is associated with the injector 10, the flange 34 is positioned into and/or in contact with the syringe mount 12 located on the forward end 40 of a housing 42 of the injector 10. The syringe mating section 32 and syringe mount 12 may be utilized to facilitate operative connection of the syringe 14 to the injector 10, as will be described in greater detail below.

The discharge tip 26 of the syringe 14 has an orifice 44 defined in its remote end, which may communicate with an internal syringe cavity 46 defined within the neck 24, the conical front wall 22, and the body 18 of the syringe 14. A rearward end 48 of the cavity 46 may be defined by a generally forward facing surface 50 of a syringe plunger 52. In the illustrated embodiment, this forward facing surface 50 is substantially conical. The surface 50 may be of a slope that conforms to the slope of the interior of the conical front wall 22. The syringe plunger 52 may be snugly slidable within the body 18 of the syringe 14 such that the cavity 46 is of variable volume. Tubing (not shown) may be operatively connected to the discharge tip 26 such that fluid can be expressed from the syringe 14 through the tubing.

Referring now to Figures 1, 4B, and 5B, the syringe plunger 52 can be seen more clearly within the body 18 of the syringe 14. When the syringe 14 is attached to the injector 10, the syringe plunger 52 is preferably located proximal to and in substantial alignment with the drive ram 16 of the injector 10. The drive ram 16 is driven by a motor (not shown) to move in a forward or rearward motion along its longitudinal axis 54 to deploy the drive ram 16, and thus to responsively deploy the syringe plunger 52 in a forward or rearward motion along the longitudinal axis 36 of the syringe 14, to inject fluid into a patient or to fill the syringe 14 with fluid, respectively. For example, one may load a prefilled syringe into the injector 10 and, by deploying the plunger 52 in a forward direction, may thereby expel fluid from the syringe 14. In so doing, the fluid may be injected into the patient. Alternatively, an empty syringe may be loaded into the injector 10 while the syringe plunger 52 may be located at or near its forward-most position. Thereafter, fluid (e.g., contrast media) may be loaded into the syringe 14 by operatively connecting the syringe 14 to a source of fluid and retracting the syringe plunger 52 in a rearward direction in order to draw fluid into the syringe 14.

The injector 10 may be designed to accommodate prefilled syringes or empty syringes of varying volumes. For example, the injector 10 may be adapted to receive 125 ml prefilled syringes (e.g., Ultraject® syringe commercially available from Mallinckrodt Inc. of St. Louis, Missouri). Such syringes may be used for injecting contrast media into a patient. These 125 ml syringes may be prefilled with any of a range of appropriate amounts of fluid, such as 50 ml, 75 ml, 100 ml, 125 ml, or other amount. Additionally, the injector 10 may accommodate an empty syringe of any of a variety of sizes (e.g., 50 ml, 75 ml, 100 ml, 125 ml, 130 ml, etc.).

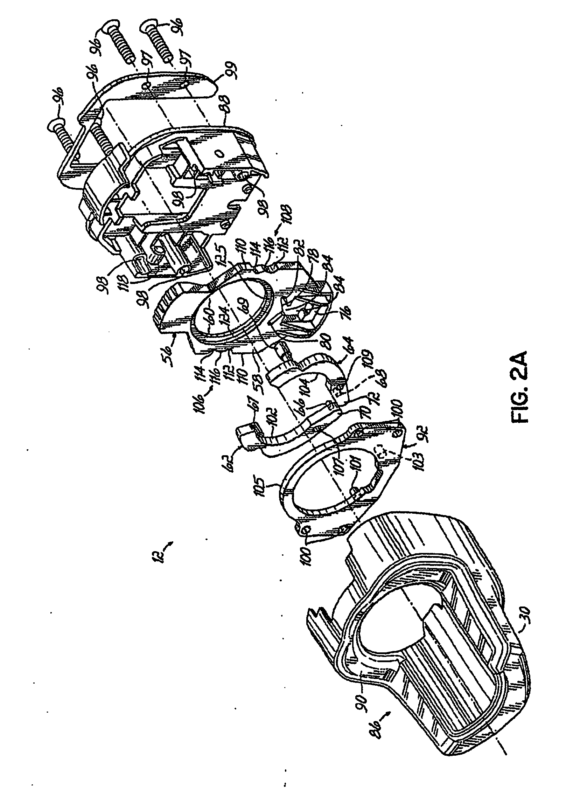

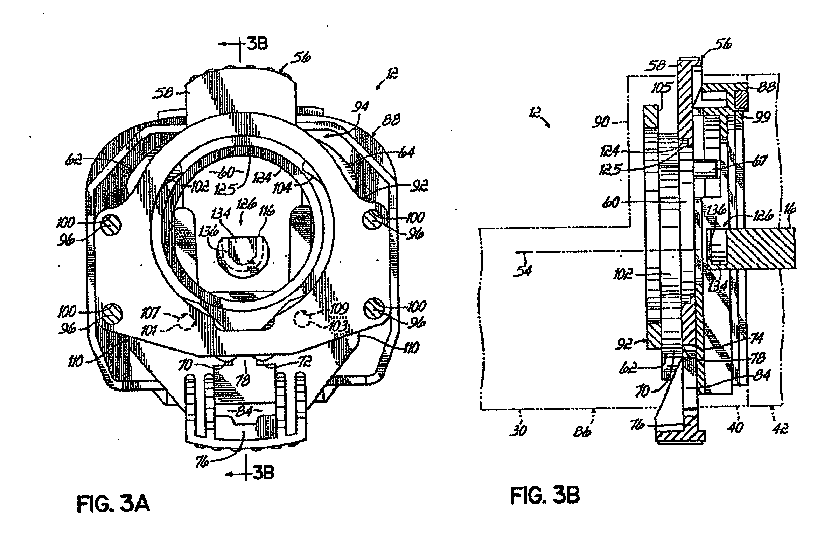

Referring now to Figures 2A-5B, one embodiment of a syringe mount 12 is shown. The syringe mount 12 includes a movable actuator 56 including a wall member 58 defining an orifice 60, and at least a first movable member 62 operatively coupled to the actuator 56 and responsively movable therewith. More specifically, the syringe mount 12 of the illustrated embodiment includes first and second movable members 62, 64 that are operatively coupled to the wall member 58 of the actuator 56. The first and second movable members 62, 64 include first and second pins 66, 68 operatively connected thereto. The first pin 66 is operatively coupled near a first end 70 of the first movable member 62, and the second pin 68 is operatively coupled near a first end 72 of the second movable member 64. The first and second pins 66, 68 are received in at least one slot 74 defined in the wall member 58 of the actuator 56, to couple the first and second movable members 62, 64 thereto. The actuator 56 is disposed proximally of the first and second movable members 62, 64. Further, the first and second members 62, 64 may include first and second rods 67, 69 projecting rearwardly therefrom. These first and second rods 67, 69 may confront and move along the outer contour of the wall member 58 of the actuator 56, as the first and second movable members 62, 64 move between open and closed positions.

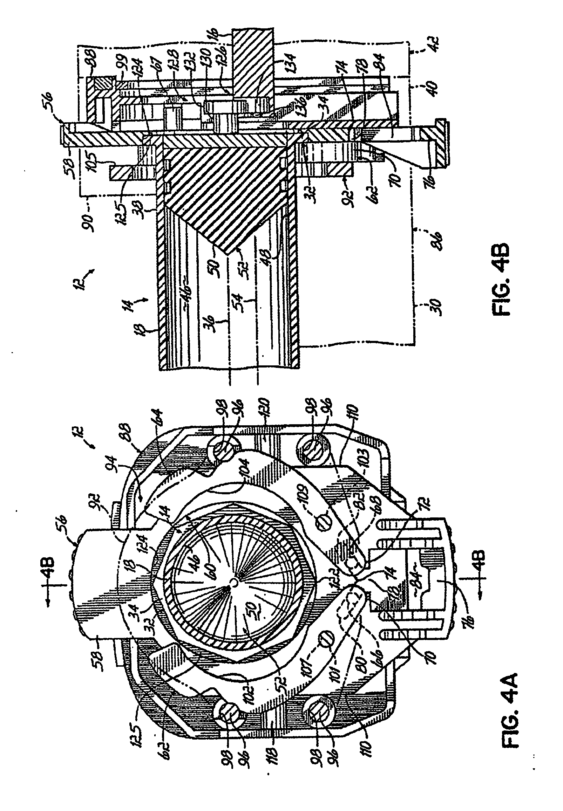

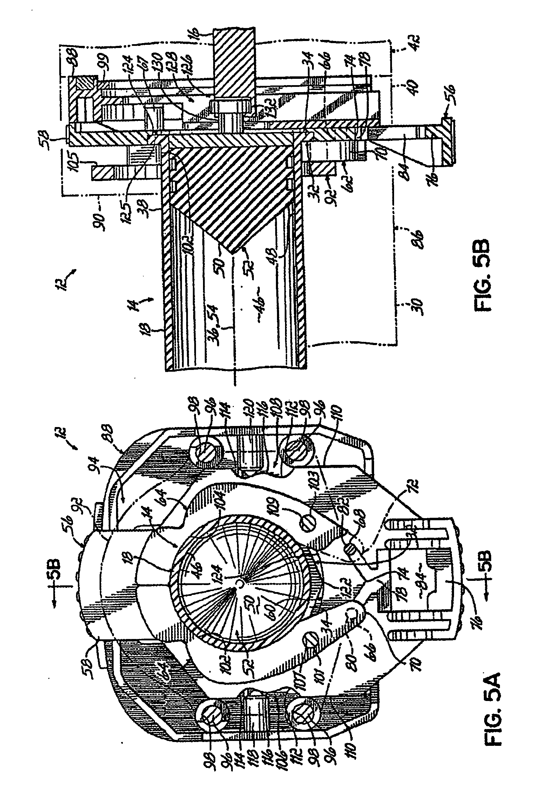

The slot 74 is defined by the wall member 58 of the actuator 56 at a base portion 76 thereof. The first and second pins 66, 68 are movable (e.g., slidable and optionally rotatable) within the slot 74. Each of the first and second pins 66, 68 can move from a position proximal to the center 78 of the slot 74, to positions near first and second terminal ends 80, 82 of the slot 74. The first and second pins 66, 68 do not both move on one side of the slot 74. Rather, the first pin 66 is adapted to move within one portion of the slot 74, and the second pin 68 is adapted to move within another portion of the slot 74. In particular, in the illustrated embodiment, a base portion 76 of the wall member 58 includes an opening 84 having a top portion thereof in a shape at least generally similar to a "V." The first and second pins 66, 68 are disposed in the "V" portion of this opening 84. When the first and second pins 66, 68 are positioned near the intersection of the two legs of the "V," the first and second movable members 62, 64 are in an open position (see Figure 4A). When the first and second pins 66, 68 are positioned near the first and second terminal ends 80, 82 of the "V," the first and second movable members 62, 64 are in a closed position (see Figure 5A). While the slot 74 of the illustrated embodiment is shown and described here as generally having a "V" shape, it will be recognized by those skilled in the art that such a "V" shape is not necessary, and any other shape can be used that allows the first and second movable members 62, 64 to move sufficiently within a slot to operatively connect a syringe to an injector 10. For example, the slot 74 may have a "U" or "C" shape. Further, those skilled in the art will recognize that more than one slot may be used. For example, two slots forming a "V" shape proximal to the base 76 of the wall member 58 can receive the first and second pins 66, 68 near the point of the "V." Again, those skilled in the art will recognize that the slots do not necessarily have to be in the shape of a "V."

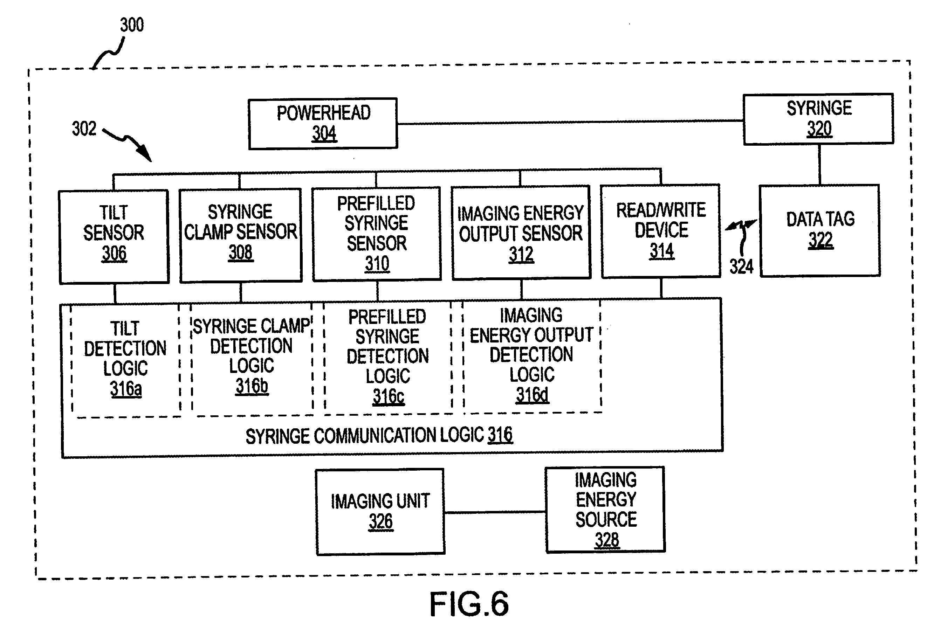

As can be seen from Figures 2A-5B, the actuator 56 and the first and second movable members 62, 64 of the syringe mount 12 are held within a face plate 86 of the housing 42 of the injector 10 (additional views of the face plate may be seen in Figures 6-12). Referring particularly to Figure 2A, the face plate 86 includes a proximal wall portion 88, a distal wall portion 90, a cradle 30 extending distally from the distal wall portion 90, and a coupling plate 92. The first and second movable members 62, 64 are located between the coupling plate 92 and the wall member 58 of the actuator 56, and all three components are then contained within an interior cavity 94 of the face plate 86, formed between the proximal wall portion 88 and distal wall portion 90. The actuator 56 and the first and second movable members 62, 64 are movable within the interior cavity 94. The coupling plate is preferably substantially immobile relative to the proximal and distal wall portions of the face plate 86, as it is preferably fixed to at least one of the proximal and distal wall portions 88, 90. In the illustrated embodiment, this fixing occurs through the use of screws 96, which extend through orifices 97 in a rear plate 99, orifices 98 in the proximal wall portion 88, orifices 100 in the coupling plate 92, and are received in orifices (not shown) in the distal wall portion 90.

The coupling plate 92 includes first and second pivoting shafts 101, 103 projecting from a proximal surface 105 thereof. These first and second pivoting shafts 101, 103 are received in first and second shaft openings 107, 109 defined in the first and second movable members 62, 64, respectively. As such, the first and second movable members 62, 64 are able to exhibit a pivoting motion about the corresponding first and second pivot shafts 101, 103. Stated another way, the first and second movable members 62, 64 are coupled with corresponding the first and second pivoting shafts 101, 103 in a manner such that the movable members 62, 64 can pivot thereabout. The first and second pivoting shafts 101, 103 thus may be said to provide pivot points for the first and second movable members 62, 64.

To initiate loading of the syringe 14 into the syringe mount 12, the flange 34 at the rearward end 38 of the syringe 14 may be passed through an aperture in each of the distal wall portion 90 of the syringe mount 12 and the coupling plate 92 and may be received into the orifice 60 defined in the actuator 56. While the rearward end 38 of the syringe 14 is located in the orifice 60, the syringe 14 may be moved in a first direction substantially perpendicular to the longitudinal axis 54 of the drive ram 16 of the injector 10. Herein, this direction will be referred to as a "downward" direction (as the motion is down relative to the injector 10). However, it will be recognized by those skilled in the art that the motion does not have to be "downward," but that the components of the syringe mount 12 can be configured such that motion in other directions can effect appropriate engagement of the syringe 14 (including, but not limited to, "upward" movement, "side-to-side" movement, or any other appropriate, substantially perpendicular movement such that the longitudinal axis 36 of the syringe 14 is moved into a substantially coaxial relationship with the longitudinal axis 54 of the drive ram 16). This downward motion, in turn, responsively moves the actuator 56 in the downward direction. The motion of the actuator 56 in the downward direction causes each of the first and second pins 66, 68 to move to the corresponding first and second ends 80, 82 of the slot 74 defined in the base portion 76 of the wall member 58. This movement of the pins 66, 68 occurs because the first and second movable members 62, 64 cannot move in the downward direction due to the first and second pivoting shafts 101, 103 of the fixed coupling plate 92 being located within the first and second shaft openings 107, 109 of the first and second movable members 62, 64. Thus, as the actuator 56 moves in the downward direction, the first and second pins 66, 68 move within the slot 74 to the first and second terminal ends 80, 82 thereof. Because the first and second movable members 62, 64 cannot move downwardly, they instead pivot about the pivot points provided by the first and second pivoting shafts 101, 103. In other words, the first and second movable members 62, 64 rotate about the corresponding first and second pivoting shafts 101, 103 at the respective first and second shaft openings 107, 109. As such, the first and second movable members 62, 64 pivot to engage (e.g., substantially, circumferentially envelop) the rearward end 38 of the syringe 14 (see Figure 5A). Since the flange 34 of the syringe 14 is located within the actuator 56 during this pivoting movement of the movable members 62, 64, the first and second movable members 62, 64 engage the body 18 of the syringe 14 (rather than the flange 34). In embodiments where the movable members 62, 64 are designed such that this engagement with the body 18 of the syringe 14 may be characterized as a substantial enveloping of the body 18, it may be said that this type of engagement allows for greater coverage of the syringe 14 than found in prior syringe mounts, and thus, potentially allows the syringe 14 to withstand greater injection pressures.

In the illustrated embodiment, the first and second movable members 62, 64 are opposite one another and are positioned about the longitudinal axis 54 of the drive ram 16. Further, the first and second movable members 62, 64 each have an arcuate face 102, 104. These arcuate faces 102, 104 are shown as being diametrically opposite one another and located exterior to the body 18 of the syringe 14. When the syringe 14 is properly engaged with the syringe mount 12 of the injector 10, the first and second movable members 62, 64 of the syringe mount 12 are in contact with the side surface of the exterior body 18 of the syringe 14 to hold the syringe 14 in place and in alignment with the drive ram 16 of the injector 10.

In some embodiments, the arcuate faces 102, 104 of the movable members 62, 64 may bear one or more types of engagement enhancing features (e.g., grooves, bumps, indentations, ridges, teeth, combinations thereof, and the like) to improve the ability of the movable members 62, 64 to grip and/or hold the syringe 14. In some embodiments, a grip enhancing coating (e.g., Santoprene® elastomer) may be applied to the arcuate faces 102, 104 of the movable members 62, 64 to facilitate gripping/holding of the syringe 14.

The pivotal movement of the first and second movable members 62, 64 alters the distance between the arcuate faces 102, 104 as they pivot toward and away from one another. In the illustrated embodiment, the first and second movable members 62, 64 are each movable. In some embodiments, it is possible to use a single movable member disposed in spaced relation to an immobile member (e.g., arcuate stop or abutment) toward which the single movable member may be moved.

In some embodiments, first and second movable members 62, 64 are not necessary for appropriate syringe engaging function. In such embodiments, a single gripping member may be used to engage the syringe 14, thereby operatively connecting the syringe 14 to the injector 10. In such embodiments, the single movable member should cover enough of the circumference of the syringe 14, when in contact with the body 18, to hold the syringe 14 against the injector 10. In such embodiments, each arm extending from a center point of the movable member may have a degree of elasticity such that the arms may splay outwardly and inwardly to allow for insertion and/or removal of the syringe 14.

The wall member 58 of the actuator 56 is shown as having a peripheral side surface 110 that includes a first undulating contour 106 and a second undulating contour 108. As shown, the second undulating contour 108 is positioned substantially opposite the first undulating contour 106. Each of these first and second undulating contours 106, 108 includes a first valley 112, a second valley 114, and a ridge 116 disposed therebetween. When positioned within the syringe mount 12 of the injector 10, these first and second undulating contours 106, 108 are confronted by first and second projections 118, 120 (see Figures 2A and 5A), which are adapted to ride along the surface of the first and second undulating contours 106, 108 as the actuator 56 is moved between the first and second positions. In the illustrated embodiment, the first and second projections 118, 120 are coupled to the proximal wall portion 88 of the face plate 86, and are spring-biased in a direction toward each of the first and second undulating contours 106, 108. The interaction of the first and second detents 118, 120 and first and second undulating contours 106, 108 assist in maintaining the actuator 56 in either the first or second position until a user desires to move the actuator 56 to either load or unload the syringe 14. In some embodiments, the first and second pins 66, 68 may include bias springs associated with each of the first and second movable members 62, 64. In such embodiments, one end of each of the bias springs may be in contact with its respectively associated movable member, and the opposite end of each bias spring may seat or bear against portions of the housing 42 (or face plate 86) of the injector 10. In some embodiments, at least a portion of these bias springs may be disposed about the pins 66, 68, which form the pivot axes of the first and second movable members 62, 64.

To load a syringe 14 into the injector 10, the syringe 14 is positioned relative to the wall member 58 of the actuator 56 such that the flange 34 at the rearward end 38 of the syringe 14 is received within the orifice 60 of the wall member 58 such that at least one contact point 122 on the periphery of the flange 34 contacts or can be brought into contact with a peripheral surface 124 defining the orifice 60. More specifically, the flange 34, in certain embodiments, may be received by a recess 125 in the actuator 56. The actuator 56 is shown in Figure 4A as being in the first position, such that the first and second movable members 62, 64 are in the open position. Also in this first position, the first and second projections 118, 120 are in contact with the first valleys 112 of the corresponding first and second undulating contours 106, 108. The force of the spring bias of the first and second projections 118, 120 at least assists in preventing the wall member 58 of the actuator 56 from moving unassisted to the second position. Further, the drive ram 16 of the injector 10 is preferably positioned such that a plunger coupling mechanism 126 is aligned with a coupling mechanism 128 extending from a rearward face of the syringe plunger 52 (see Figure 4B).

A user then applies a force to the syringe 14 in a direction substantially perpendicular to, and towards, the longitudinal axis 54 of the drive ram 16. The flange 34 of the syringe 14, contacting the peripheral surface 124 of the wall member 58, is utilized to force the wall member 58 of the actuator 56 to responsively move in a direction substantially perpendicular to the longitudinal axis 54 of the drive ram 16. Enough force is applied to overcome the spring-bias of the first and second projections 118, 120, such that the actuator 56 moves from the first position to the second position. As this occurs, the first and second projections 118, 120 ride along the first and second undulating contours 106, 108 from the first valleys 112, along the ridges 116, and into the second valleys 114. The first and second projections 118, 120 may then be utilized to at least assist in maintaining the wall member 58 in the second position shown in Figure 5A.

The movement of the wall member 58 from the first position to the second position cooperatively moves the slot 74 of the wall member 58 in a direction substantially perpendicular to the longitudinal axis 54 of the drive ram. And thus, the slot 74 moves relative to the first and second pins 66, 68, thereby causing the first and second pins 66, 68 to move relative to and within the slot 74. More specifically, in the illustrated embodiment, the first and second pins 66, 68 move within the V-shaped slot from a position proximal to the point of the "V," to positions proximal to the terminal ends of each leg of the "V" (from the position shown in Figure 4A, to the position shown in Figure 5A). This movement causes a responsive pivotal movement of the first and second movable members 62, 64 from the open position to the closed position such that the rearward end 38 of the syringe 14 is engaged by the first and second movable members 62, 64. In particular, as the actuator 56 moves in the downward direction, the first and second pins 66, 68 move within the slot 74 to the first and second terminal ends 80, 82 thereof. Because the first and second movable members 62, 64 cannot move downwardly, they instead pivot about the pivot points provided by the first and second pivoting shafts 101, 103. In other words, the first and second movable members 62, 64 rotate about the first and second pivoting shafts 101, 103 at the first and second shaft openings 107, 109, respectively.

As the wall member 58 is moved from the first position to the second position, and the syringe 14 moves with the wall member 58 from a position not engaged by the movable members 62, 64 to a position engaged by the movable members 62, 64, the coupling mechanism 128 at the rearward end 38 of the syringe plunger 52 moves from a position not engaged with the plunger coupling mechanism 126 of the drive ram 16 to a position engaged with the plunger coupling mechanism 126 of the drive ram 16. In the illustrated embodiment (see Figures 4B and 5B), when the flange 34 of the syringe 14 is aligned with the orifice 60 defined by the wall member 58, the syringe plunger 52 within the syringe 14 is preferably positioned such that the coupling mechanism 128 on the rearward face of the syringe plunger 52 is aligned with the plunger coupling mechanism 126 of the drive ram 16. The coupling mechanism 128 of the illustrated syringe plunger 52 is a projection 128 extending from the rearward face of the syringe plunger 52. This projection 128 may be characterized as exhibiting a "T" shape having a stem portion 130 (parallel to the longitudinal axis 36 of the syringe 14) topped by a cap portion 132 (transverse to the longitudinal axis of the syringe 14). As the wall member 58 is moved from the first position to the second position, the cap portion 132 of the coupling mechanism 128 may be received by the plunger coupling mechanism 126, which in the illustrated embodiment, is a slot 134 formed in the forward end of the drive ram 16.

A slot 134 is defined in the forward end of the drive ram 16 in a shape to receive the coupling mechanism 128 of the syringe 14, and particularly the cap portion 132 thereof. A cross-section of the plunger coupling element 126 is shown as exhibiting a J-shape (having a slot within a hook portion of the "J" configured to receive the cap portion 132), such that when the syringe plunger 52 is engaged with the drive ram 16, the distal end 136 of the "J" shape is positioned distally of a part of the cap portion 132 of the coupling mechanism 128. Thus, when the syringe 14 is initially inserted into the actuator 56 (in the first position), the cap portion 132 of the coupling mechanism 128 is "above" the plunger coupling element 126 of the drive ram 16. However, as the actuator 56 is moved to the second position, the cap portion 132 of the coupling mechanism 128 is moved to be positioned proximally of the distal end 136 of the plunger coupling mechanism 126 of the drive ram 16. Once engaged, an injection procedure may be run, such as by translating the drive ram 16 forward along its longitudinal axis 54 to dispense a fluid, such as contrast media, from the syringe 14. While the slot 134 and extension 128 of the illustrated embodiment have shapes referred to herein as "J" and "T," respectively, it will be recognized by those of skill in the art that any shape that facilitates coupling may be used. Additionally, while the illustrated embodiment depicts first a coupling mechanism 128 and plunger coupling mechanism 126 that result in a passive coupling, those of skill in the art will recognize that coupling mechanisms and plunger coupling mechanisms that result in an active coupling (one which involves some degree of positive gripping) may be used.

As described previously, the syringe mount 12 allows for the syringe 14 to be removed from the face plate 86 and/or forward end 40 of the injector 10, when the drive ram 16 of the injector 10 is at any position. It does not require the drive ram 16 to be returned to a "home" position before detaching the syringe 14 from the injector 10. Thus, during an injection procedure, the translation of the drive ram 16 may be stopped while the drive ram 16 is in an extended position from the front face place 86 of the injector 10. A user can then grip the syringe 14 and move it in an upward direction, thereby overcoming the spring-biased force of the first and second projections 118, 120 to cause the actuator 56 to move from the second position to the first position. As this occurs, the first and second projections 118, 120 ride along the first and second undulating contours 106, 108 from the second valleys 114, over the ridges 116, and into the first valleys 112. Simultaneously, the first and second pins 66, 68 of the first and second movable members 62, 64 will move within the V-shaped slot of the wall member 58 from a position near the terminal ends 80, 82 of the arms of the V to a position near the point of the V. This causes the first and second movable members 62, 64 to pivot from the closed position to the open position by pivoting about the pivot points created by the interaction of the first and second pivoting shafts 101, 103 with the first and second shaft openings 107 109. Due to the positioning of the flange 34 at the rearward end 38 of the syringe 14 within the orifice 60 of the actuator 56, the actuator 56 allows for enough vertical syringe movement for the T-shaped coupling mechanism on the rearward face of the syringe 14 to clear the slot on the forward end of the drive ram 16, thereby allowing removal of the syringe 14 from the injector 10.

The power injectors 210, 10 of Figures 1A and 1B each may be used for any appropriate application, including without limitation for medical imaging applications where fluid is injected into a subject (e.g., a patient). Representative medical imaging applications for the power injectors 210, 10 include without limitation computed tomography or CT imaging, magnetic resonance imaging or MRI, SPECT imaging, PET imaging, X-ray imaging, angiographic imaging, optical imaging, and ultrasound imaging. The power injectors 210, 10 each could be used alone or in combination with one or more other components. The power injectors 210, 10 each may be operatively interconnected with one or more components, for instance so that information may be conveyed between the power injector 210, 10 and one or more other components (e.g., scan delay information, injection start signal, injection rate).

Any number of syringes may be utilized by each of the power injectors 210, 10, including without limitation single-head configurations (for a single syringe) and dual-head configurations (for two syringes). In the case of a multiple syringe configuration, each power injector 210, 10 may discharge fluid from the various syringes in any appropriate manner and according to any timing sequence (e.g., sequential discharges from two or more syringes, simultaneous discharges from two or more syringes, or any combination thereof). Multiple syringes may discharge into a common conduit (e.g., for provision to a single injection site), or one syringe may discharge into one conduit (e.g., for provision to one injection site) while another syringe may discharge into a different conduit (e.g., for provision to a different injection site). Each such syringe utilized by each of the power injectors 210, 10 may include any appropriate fluid, for instance contrast media, a radiopharmaceutical, saline, and any combination thereof. Each such syringe utilized by each of the power injectors 210, 10 may be installed in any appropriate manner (e.g., rear-loading configurations may be utilized; front-loading configurations may be utilized; side-loading configurations may be utilized).

An embodiment of an imaging system is illustrated in Figure 6 and is identified by a reference numeral 300. Two primary components of the imaging system 300 are schematically illustrated - an imaging unit 326 and a power injector 302. The imaging unit 326 may be of any appropriate size, shape, configuration, and/or type, and includes at least one imaging energy source 328. In one embodiment, the imaging unit 326 is in the form of an MR or MRI scanner (magnetic resonance), which may utilize an arrangement of typically high-frequency coils (e.g., three coils that provide three orthogonal gradients in the x, y, and z directions of the scanner) as one imaging energy source 328 (e.g., to create a strong magnetic field) and an RF transmission system as another imaging energy source 328 (e.g., to transmit RF signals that rotate the magnetic field). The output of the imaging energy source(s) 328 is what facilitates the acquisition of a medical image (e.g., of an anatomical/biological structure).

The power injector 302 includes a powerhead 304. At least one syringe 320 may be installed on the powerhead 304 in any appropriate manner, and when installed may be considered to be part of the power injector 302. At least one RFID tag 322 is integrated with the syringe 320 in any appropriate manner and at any appropriate location. Multiple RFID tags 322 could be disposed in any appropriate arrangement on the syringe 320. Any appropriate information may be stored on each syringe RFID tag 322 and any appropriate number of RFID tags 322 may be utilized. Other data storage device types may be appropriate for the syringe 320.

The power injector 302 also includes a syringe communication module or logic 316. Generally, the syringe communication logic 316 will be described as that which monitors, analyzes, or otherwise assesses data from one or more sources to determine whether a syringe communication (e.g., read/write operation) should be initiated. Any component or combination of components that analyzes data from one or more sources to determine whether a communication should be initiated between an RFID read/write device 314 and a syringe RFID tag 322 will be considered to be at least part of the syringe communication logic 316 for purposes of the power injector 302 and the medical imaging system 300.

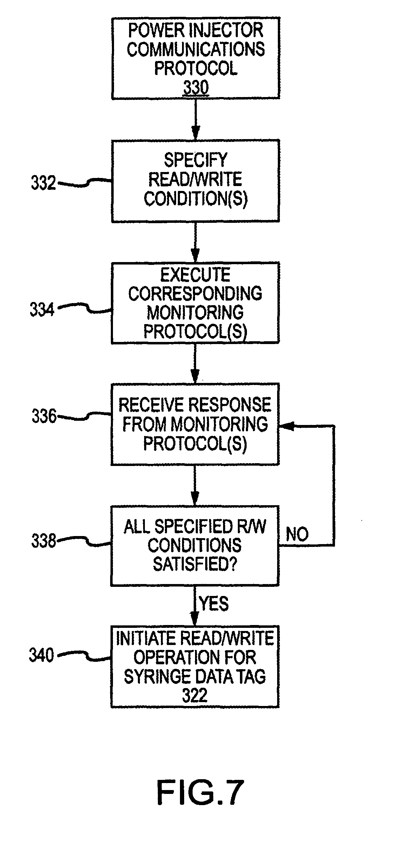

The syringe communication logic 316 is operatively interconnected with an RFID read/write device 314 (more generally, a communication device) of any appropriate size, shape, configuration, and/or type (e.g., a powered antenna). The RFID read/write device 314 is part of the power injector 302. Generally, the syringe communication logic 316 may be configured to control the timing of communications between this RFID read/write device 314 and one or more RFID tags 322 on a syringe 320 installed on the powerhead 304 over any appropriate communications link 324. Data may be acquired by the syringe communication logic 316 from various sources, where this data is used to make a communication determination (e.g., whether to initiate a read and/or write operation) in relation to the RFID read/write device 314. For instance, the syringe communication logic 316 may communicate in any appropriate manner with any one or more of the following, and each of which may be of any appropriate size, shape, configuration, and/or type: a tilt sensor 306; a syringe clamp sensor 308; a prefilled syringe sensor 310; and an imaging energy output sensor 312. The functionality associated with the syringe communication logic 316 being in communication with each these components will be addressed in more detail below in relation to the protocols of Figures 7-11B.

The tilt sensor 306 may provide data for determining if the power injector 302 is experiencing at least some type of change in orientation (e.g., an orientation of the powerhead 304 and/or a syringe 320 installed on the powerhead 304). The tilt sensor 306 could provide information on the magnitude of an orientational change, the direction of an orientational change, the time over which an orientational change occurred, or any combination thereof. In one embodiment, the tilt sensor 306 is in the form of an accelerometer incorporated by the powerhead 304. Any appropriate number of tilt sensors 306 could be utilized to provide the noted function, and each such tilt sensor 306 may be disposed at any appropriate location of the power injector 302. In the case of the power injector 10 discussed above in relation to Figure 1B, the tilt sensor 306 may determine if the powerhead 42 is being tilted, rotated, or moved at least generally about the axis 43.

The data acquired by the tilt sensor 308 may be analyzed for the existence of a predetermined condition by the syringe communication logic 316. Part of the syringe communication logic 316 could be dedicated to this analysis, such as tilt detection logic 316a. In the case where the tilt sensor 306 is in the form of an accelerometer, the syringe communication logic 316 may be monitoring a magnitude of the acceleration, a direction or vector of the acceleration, or both. In any case, once the syringe communication logic 316 has determined that all requirements for an orientation change of the power injector 302 have been met, the syringe communication logic 316 may initiate communication between the RFID read/write device 314 and at least one syringe RFID tag 322.

A syringe clamp may be incorporated by the powerhead 304 to hold/restrain a syringe 320 in at least one dimension when the syringe clamp is in its closed configuration (e.g. to limit or restrain movement of the syringe 320 in a plane that is orthogonal to an axis along which its syringe plunger is able to move in at least one direction), for instance by the syringe clamp extending about at least substantially the entire perimeter of the barrel of the syringe 320, although such may not be required by all syringe clamp configurations. The power injector 302 may use a syringe clamp of any appropriate size, shape, configuration, and/or type. A representative syringe clamp is addressed above in relation to the power injector 10 of Figures 1B-5B, and is collectively defined by the members 62 and 64 shown in Figure 2A and certain other figures.

The syringe clamp sensor 308 utilized by the power injector 302 of Figure 6 may provide a function of determining or detecting if a syringe clamp of the power injector 302 has been moved into/through one or more predefined/predetermined states, configurations, or positions (e.g., if the syringe clamp has been moved into an open state or configuration; if the syringe clamp has been moved into a closed state or configuration; if the syringe clamp has been moved into/through a certain intermediate state or configuration between its open and closed states/configurations). The syringe clamp sensor 308 may provide this detection functionality in any appropriate manner. Any appropriate number of syringe clamp sensors 308 may be used to monitor each syringe clamp, including a single syringe clamp sensor 308 or multiple syringe clamp sensors 308 disposed in any appropriate arrangement.

Representative embodiments for the syringe clamp sensor 308 include without limitation a magnet/Hall Effect sensor combination, optical electronics, electro-mechanical switches, inductive proximity sensors, and potentiometers. An optical emitter/detector pair (optical electronics) may be arranged such that when the light path between the pair is interrupted by a movement of the syringe clamp into/through a certain position, the detector may send an appropriate signal (directly or indirectly) to the syringe communication logic 316. An electro-mechanical switch may be positioned such that the syringe clamp will move into contact with and/or land on the electro-mechanical switch when the syringe clamp is moved into/through a certain position. When such an electro-mechanical switch is activated (e.g., depressed) in response to such a movement of the corresponding syringe clamp, the switch may send an appropriate signal (directly or indirectly) to the syringe communication logic 316. An inductive proximity sensor may be positioned such that the syringe clamp will move into communication range with the proximity sensor when the syringe clamp is moved into/through a certain position. Inductive proximity sensors are non-contact devices that set up a radio frequency field. The presence of a metallic object alters this field, and the proximity sensor is able to detect this alteration. The proximity sensor may send an appropriate signal (directly or indirectly) to the syringe communication logic 316. Any such proximity sensor could be digital (i.e., on or off) or analog. Reading analog sensor values would allow software to translate a multitude of syringe clamp positions with one or more proximity sensors.

The data acquired by the syringe clamp sensor 308 may be analyzed for the existence of a predetermined condition by the syringe communication logic 316. Part of the syringe communication logic 316 could be dedicated to this analysis, such as syringe clamp detection logic 316b. The analysis in this case may simply be the existence or lack of a signal. In any case, once the syringe communication logic 316 has determined that the syringe clamp has moved into/through a certain configuration or position, the syringe communication logic 316 may initiate communication between the RFID read/write device 314 and at least one syringe RFID tag 322.

A prefilled syringe sensor 310 may provide data for determining if a prefilled syringe 320 has been installed on the powerhead 304 of the power injector 302. In one embodiment, the prefilled syringe sensor 310 is in the form of a transmitter antenna, along with one or more receiver antennas. The transmitter antenna may transmit an RF signal of a known strength. A receiver antenna may receive this signal from the transmitter antenna. The receiver antenna is positioned relative to the transmitter antenna such that the signal from the transmitter antenna will pass through a zone that would be occupied by a prefilled syringe 320 when installed on the powerhead 304 (e.g., a syringe zone or prefilled syringe zone).

The data acquired by the prefilled syringe sensor 308 may be analyzed for the existence of a predetermined condition by the syringe communication logic 316. Part of the syringe communication logic 316 could be dedicated to this analysis, such as prefilled syringe detection logic 316c. In any case, this analysis generally entails making a determination as to whether or not there has been at least a certain attenuation of the signal (as received after passing through a prefilled syringe zone) compared to the signal originally sent. Although the transmitter could be operatively interconnected with the prefilled syringe detection logic 316c, one or more characteristics of the signal to be sent by the transmitter may be stored in memory or may be otherwise made available to the prefilled syringe detection logic 316c for purposes of assessing signal attenuation.

The syringe communication logic 316 may be configured to have a comparative field strength value that is associated with the condition when a prefilled syringe 320 is not installed on the powerhead 304. Therefore, a certain change in the field strength, measured by the receiver antenna, in relation to the comparative field strength value, may indicate that a prefilled syringe 320 has been installed on the powerhead 304. Generally, the liquid in the prefilled syringe 320 should noticeably attenuate the signal from the transmitter antenna, and this attenuation may be detected by the receiver antenna and may be associated with a condition of a prefilled syringe 320 being installed on the powerhead 304.

An imaging energy output sensor 312 may be utilized to acquire data for determining if the imaging unit 326 (more specifically one or more of its imaging energy sources 328) is being operated to acquire a medical image. The imaging energy output sensor 312 is not simply obtaining a control signal from the imaging unit 326. Instead, the imaging energy output sensor 312 is monitoring the environment to identify when at least one imaging energy source 328 is being operated for image acquisition purposes. The imaging energy output sensor 312 may be of any appropriate size, shape, configuration, and/or type. In one embodiment, the RFID read/write device 314 actually provides the function of the imaging energy output sensor 312, such that the imaging energy output sensor 312 and the RFID read/write device 314 are the same, common structure. However, a separate imaging energy output sensor 312 and RFID read/write device 314 could be utilized and as shown in Figure 6.

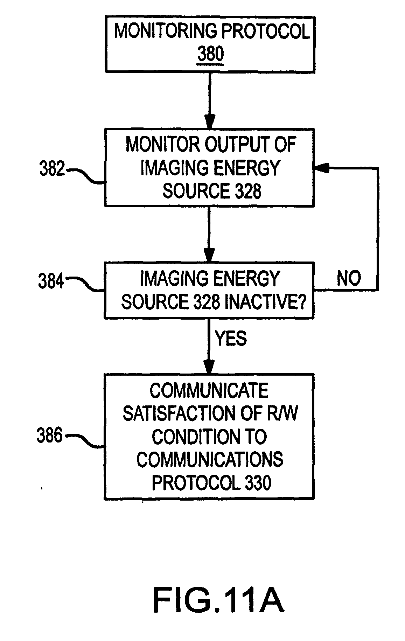

The data acquired by the imaging energy output sensor 312 may be analyzed for the existence of a predetermined condition by the syringe communication logic 316. Part of the syringe communication logic 316 could be dedicated to the analysis, such as imaging energy output detection logic 316d. Representative ways in which this analysis may be undertaken will be discussed in more detail below in relation to the monitoring protocols of Figures 11A-B. However, generally the analysis could simply entail analyzing a signal received from the imaging energy output sensor 312 to determine if it is above a certain threshold, analyzing a signal from the imaging energy output sensor 312 to identify a pattern by which an imaging energy source 328 is being cycled between inactive and active states, or the like.

Communication between the RFID read/write device 314 and one or more RFID tags 322 on the syringe 320 may be triggered by an output from one or more of the sensors 306, 308, 310, and 312 used by the power injector 302 of Figure 6. One embodiment of a power injector communications protocol is illustrated in Figure 7 and is identified by a reference numeral 330. A condition or combination of conditions may be specified in step 332 of the protocol 330 that will trigger a communication between the RFID read/write device 314 and at least one RFID tag 322 on a syringe 320 installed on the powerhead 304. These may be referred to as syringe communication trigger conditions or as read/write conditions as shown in Figure 7. In any case, triggering a communication between the RFID read/write device 314 and at least one syringe RFID tag 322 may be based upon an output from the tilt sensor 306, the syringe clamp sensor 308, the prefilled syringe sensor 310, or the energy output sensor at 312, individually or in any combination.

It should be appreciated that the power injector 302 of Figure 6 may include any one or more of the sensors 306, 308, 310, and 312 for purposes of the syringe communication logic 316, namely to trigger a communication between the RFID read/write device 314 and a syringe RFID tag 322. Moreover, step 332 may entail specifying a single read/write condition (e.g., from a single one of the sensors 306, 308, 310, 312), or may entail specifying multiple read/write conditions (e.g., from two or more of the sensors 306, 308, 310, 312). The specification of step 332 may be executed through a graphical user interface or the like, where operations personnel would be allowed to input the desired read/write condition(s). The specification of step 332 could also be a "hard-wired" configuration for the power injector 302 - the read/write conditions could be set up prior to delivery of the power injector 302 to the end-use facility in this type of case. Another option would be for the read/write condition(s) of step 332 to be specified through a service mode or the like for the power injector 302, where only certain personnel have access to step 332.

The power injector 302 could include any two or more of the sensors 306, 308, 310, and 312. The syringe communication logic 316 could be configured to trigger a communication between the RFID read/write device 314 and at least one syringe RFID tag 322: 1) upon receipt of an appropriate signal from any one of the sensors 306, 308, 310, and 312 being utilized by the power injector 302; 2) upon receipt of an appropriate signal from two or more of the sensors 306, 308, 310, and 312 being utilized by the power injector 302; or 3) upon receipt of an appropriate signal from each of the sensors 306, 308, 310, and 312 being utilized by the power injector 302.

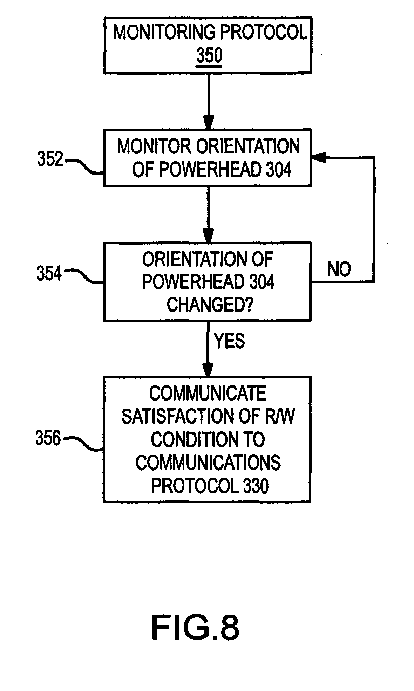

Referring back to the power injector communications protocol 330 of Figure 7, step 334 is directed to executing the monitoring protocol associated with each of the read/write conditions specified in step 332. Representative monitoring protocols that may be utilized by step 334 will be addressed below in a discussion of Figures 8-11B. Responses from the monitoring protocol(s) associated with step 334 are provided to step 336 of the power injector communications protocol 330. Step 338 monitors the receipt of responses from the monitoring protocol(s) of step 334, and when a response has been received in relation to each specified read/write condition of step 332, control passes to step 340 of the power injector communications protocol 330. Step 340 of the power injector communications protocol 330 triggers or initiates a communication between the RFID read/write device 314 and at least one syringe RFID tag 322 (e.g., at least one of a read and write operation). Generally, it is desirable for communication between the RFID read/write device 314 and a syringe RFID tag 322 to occur at a time when one or more of the imaging energy sources 328 of the imaging unit 326 is in an inactive state or mode (e.g., where the output is less than a certain threshold, including where there is no output from an imaging energy source 328 that would facilitate acquisition of a medical image). Operation of an imaging energy source 328 to acquire a medical image may adversely affect the communication between the RFID read/write device 314 and a syringe RFID tag 322,or vice versa.

Figure 8 illustrates one embodiment of a monitoring protocol 350 that may utilize an output of the tilt sensor 306 from the power injector 302 of Figure 6. An orientation relating to the powerhead 304 (e.g., the orientation of the powerhead 304 itself; the orientation of a syringe 320 installed on the powerhead 304) is monitored through execution of step 352 of the monitoring protocol 350 of Figure 8 (e.g., monitoring an output from the tilt sensor 306). Step 354 is directed to determining if the orientation of the powerhead 304 has changed in a predetermined manner. In one embodiment, this predetermined change is a minimum acceleration in a certain direction. In any case, steps 352 and 354 will continue to be executed until a predetermined change in the orientation of the powerhead 304 has been detected, at which time the protocol 350 proceeds to step 356. When a predetermined change relating to the orientation of the powerhead 304 has been identified through execution of steps 352 and 354, control passes to step 356. Step 356 of the protocol 350 sends an appropriate communication to the power injector communications protocol 330 of Figure 7 (e.g., to step 336 of the protocol 330). This communication may be characterized as a satisfaction of a read/write condition for purposes the power injector communications protocol 330 of Figure 7.

Any appropriate "predetermined orientation change" may be utilized for purposes of step 354 of the monitoring protocol 350 of Figure 8 (e.g., in order for the monitoring protocol 350 to proceed from step 354 to step 356). Representative "predetermined orientation changes" for purposes of step 354 include without limitation: 1) the powerhead 304 moving through a minimum angle in any direction or only in a specified direction; and 2) a movement of the powerhead 304, in any direction or only in a specified direction, over a certain amount of time. In one embodiment, the orientation change that will have the protocol 350 proceed from step 354 to step 356 is when the powerhead 304 is in a "tilted up" configuration - so a syringe 320 (more specifically its discharge nozzle) is projecting at least generally upwardly (e.g., above horizontal). This is a common position for the powerhead 304 when loading a fluid into a syringe 320, for purging air from a syringe 320, or the like. When the powerhead 304 is in this "tilted up" position, each imaging energy source 328 of the imaging unit 326 should be in an inactive state or mode, and therefore communications between the RFID read/write device 314 and a syringe RFID tag 322 should not be adversely affected by operation of any imaging energy source 328 of the imaging unit 326.

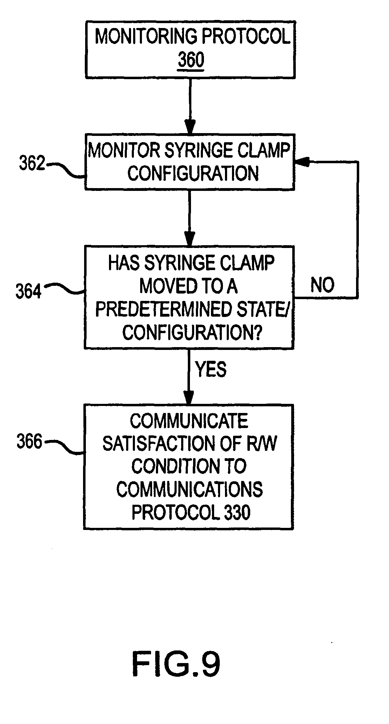

Figure 9 illustrates one embodiment of a monitoring protocol 360 that may utilize an output of the syringe clamp sensor 308 from the power injector 302 of Figure 6. The syringe clamp positional state or configuration is monitored through execution of step 362 of the monitoring protocol 360 of Figure 9 (e.g., via monitoring an output from the syringe clamp sensor 308). Step 364 is directed to determining if the syringe clamp has moved into or through a predetermined state, configuration, or position. Steps 362 and 364 will continue to be executed until the syringe clamp has been identified as moving into or through a predetermined state, configuration, or position, at which time the protocol 360 proceeds to step 366. Step 366 of the protocol 360 then sends an appropriate communication to the power injector communications protocol 330 of Figure 7 (e.g., to step 336 of the protocol 330). This communication may be characterized as a satisfaction of a read/write condition for purposes the power injector communications protocol 330 of Figure 7.

Any appropriate predetermined state or configuration for the syringe clamp may be used to trigger proceeding from step 364 to step 366 of the monitoring protocol 360 of Figure 9, for instance when the syringe clamp has been moved into a closed state/configuration, has been moved into an open state/configuration, or an intermediate state/configuration. It may be desirable to trigger communication between the RFID read/write device 314 and a syringe RFID tag 322 when the syringe clamp has been moved into a closed state/configuration (e.g., such that a syringe 320 is now installed on the powerhead 304). This would typically occur in the preparation or preliminary stages of a medical imaging procedure, and in any case well before operation of any imaging energy source 328 of the imaging unit 326 is undertaken to acquire a medical image. That is, each imaging energy source 328 of the imaging unit 326 should be in an inactive state or mode for some time after a syringe 320 has been installed on the powerhead 304.

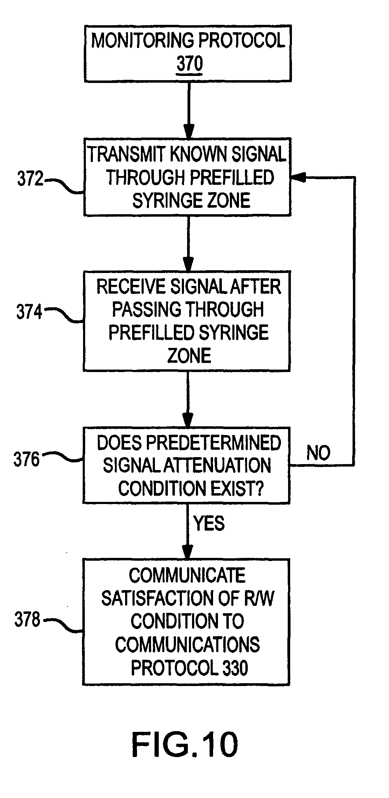

Figure 10 illustrates one embodiment of a monitoring protocol 370 that may utilize an output of the prefilled syringe sensor 310 from the power injector 302 of Figure 6. A signal of a known strength (e.g., an RF signal) may be transmitted through a zone that would be occupied by a prefilled syringe 320 when installed on the powerhead 304 (e.g., a syringe zone or a prefilled syringe zone), all pursuant to step 372 of the monitoring protocol 370. Step 374 monitors this signal after having passed through the syringe zone. The signal that is originally transmitted (step 372) is compared with the signal that is received (step 374) at step 376 to determine if there has been at least a certain amount of signal attenuation. Signal attenuation may be assessed is any appropriate manner for purposes of step 376. Steps 372, 374, and 376 should continue to be executed so long as a prefilled syringe 320 has not been installed on the powerhead 304. In this condition, there should be little to no attenuation of the signal as transmitted (step 372) compared to the signal as received (step 374). However, when a prefilled syringe 320 is installed on the powerhead 304, the transmitted signal (step 372) should be noticeably attenuated by the contents of the prefilled syringe 320 (e.g., the strength of the signal received at step 374 should be noticeably less than the strength of the signal transmitted by step 372), at which time the protocol 370 will proceed to step 378. That is, step 376 may be characterized as determining when there has been at least a certain amount of attenuation of the signal from the time of its transmission (step 372). When there is at least a certain amount of signal attenuation, the assumption is made that a prefilled syringe 320 has been installed on the powerhead 304, and as such step 378 of the protocol 370 may send an appropriate communication to the power injector communications protocol 330 of Figure 7 (e.g., to step 336 of the protocol 330). This communication may be characterized as a satisfaction of a read/write condition for purposes the power injector communications protocol 330 of Figure 7.

Syringes 320 are typically installed on the powerhead 304 of the power injector 302 in the preparation or preliminary stages of a medical imaging procedure, and in any case well before any operation of the imaging unit 326 to acquire a medical image. Therefore, triggering a communication between the RFID read/write device 314 and at least one syringe RFID tag 322 when or shortly after a determination has been made that a prefilled syringe 320 has been installed on the powerhead 304 should result in this communication being made without any risk of interference from operation of any imaging energy source 328 to acquire a medical image.

Another monitoring protocol is illustrated in Figure 11A, is identified by reference numeral 380, and may be utilized by the power injector communications protocol 330 of Figure 7. The output of at least one imaging energy source 328 is monitored through execution of step 382 of the monitoring protocol 380 of Figure 11A. Once again, a separate imaging energy output sensor 312 could be utilized by the power injector 302 for purposes of step 382, or the RFID read/write device 314 could providing this monitoring function. In any case, step 382 of the protocol 380 may be characterized as being directed to monitoring the environment in which the power injector 302 is located to determine if the imaging unit 326 is being operated in a manner so as to acquire a medical image. If a determination is made that at least one imaging energy source 328 is being operated to acquire a medical image, the monitoring protocol 380 is configured so as to not trigger communication between the RFID read/write device 314 and a syringe RFID tag 322 at this time (steps 382 and 384 of the monitoring protocol 380 will be repeated in this instance). Step 384 may simply entail comparing a signal that is received by the RFID read/write device 314 with an appropriate baseline or standard. The protocol 380 will proceed from step 384 to step 386 once a determination is made that at least one imaging energy source 328 of the imaging unit 326 is not being operated to acquire a medical image. Step 386 of the protocol 380 may send an appropriate communication to the power injector communications protocol 330 of Figure 7 (e.g., to step 336 of the protocol 330). This communication may be characterized as a satisfaction of a read/write condition for purposes the power injector communications protocol 330 of Figure 7.

Typically an imaging energy source 328 of the imaging unit 326 will remain in an "off" state, mode, or condition for more than a sufficient amount of time to allow for a communication between the RFID read/write device 314 and at least one syringe RFID tag 322. A number of configurations may be used for step 384. Once a determination has been made that at least one imaging energy source 328 is in an inactive state/mode, the protocol 380 may be configured to immediately proceed to step 386. Another option is for step 384 to be configured to not proceed to step 386 until a determination has been made that at least one imaging energy source 328 has been in an inactive state/mode for a specified amount of time.

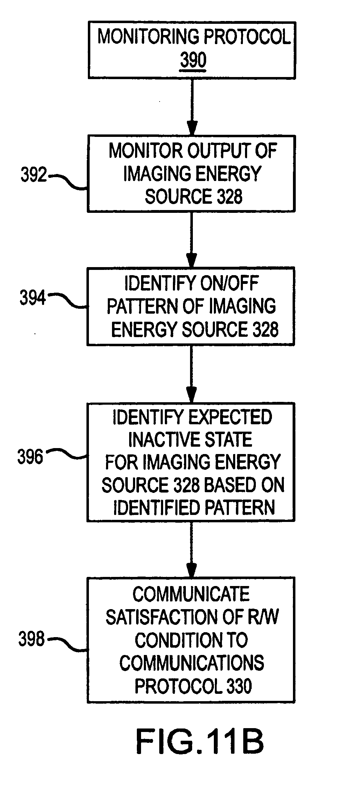

Figure 11B presents another option to trigger communications between the RFID read/write device 314 and at least one syringe RFID tag 322 for the power injector 302 of Figure 6 based upon monitoring the output of at least one imaging energy source 328 of the imaging unit 326. The output of at least one imaging energy source 328 is monitored through execution of step 392 of a monitoring protocol 390 that is illustrated in Figure 11B. Once again, a separate imaging energy output sensor 312 could be utilized by the power injector 302 for purposes of step 392, or the RFID read/write device 314 could provide this monitoring function. In any case, step 392 of the protocol 390 is directed to monitoring the environment in which the power injector 302 is located and with regard to the operation of at least one imaging energy source 328 of the imaging unit 326.

Many medical imaging procedures will cycle an imaging energy source 328 between "on" and "off" states or modes in accordance with a certain pattern (e.g., active and inactive states/modes). Step 394 of the monitoring protocol 390 attempts to identify this pattern by monitoring the output from at least one imaging energy source 328 through execution of step 394. Once this pattern is recognized by the monitoring protocol 390, step 396 may be used to identify an expected inactive state or mode for at least one imaging energy source 328. At a time when at least one imaging energy source 328 should be in an inactive state/mode according to the pattern identified by step 394, the protocol 390 proceeds from step 396 to step 398. Step 398 sends an appropriate communication to the power injector communications protocol 330 of Figure 7 (e.g., to step 336 of the protocol 330). This communication may be characterized as a satisfaction of a read/write condition for purposes the power injector communications protocol 330 of Figure 7.

The syringe communication logic 316, the tilt detection logic 316a, the syringe clamp detection logic 316b, the prefilled syringe detection logic 316c, and the imaging energy output detection logic 316d each may be implemented in any appropriate manner, including without limitation in any appropriate software, firmware, or hardware, using one or more platforms, using one or more processors, using memory of any appropriate type, using any single computer of any appropriate type or a multiple computers of any appropriate type and interconnected in any appropriate manner, or any combination thereof. The syringe communication logic 316, the tilt detection logic 316a, the syringe clamp detection logic 316b, the prefilled syringe detection logic 316c, and the imaging energy output detection logic 316d may be implemented at any single location or at multiple locations that are interconnected in any appropriate manner (e.g., via any type of network).

The foregoing description of the present invention has been presented for purposes of illustration and description. Furthermore, the description is not intended to limit the invention to the form disclosed herein. Consequently, variations and modifications commensurate with the above teachings, and skill and knowledge of the relevant art, are within the scope of the present invention. The embodiments described hereinabove are further intended to explain best modes known of practicing the invention, which is defined in the claims.

## Claims

1. A power injector comprising:
a syringe plunger driver comprising a motorized drive source (304);
an orientation sensor (306); and
a communication device (314) comprising an RFID read/write device; **characterized in that** said injector further comprises syringe communication logic (316) operatively interconnected with said orientation sensor and configured to initiate a syringe communication in response to said syringe communication logic identifying an occurrence of a first condition utilizing an output of said orientation sensor, wherein said communication device is operatively interconnected with said syringe communication logic, wherein said first condition is when said power injector experiences a predetermined change in orientation, and wherein said syringe communication is a communication between said communication device and a syringe (320) that is mounted on the power injector and that has an integrated RFID tag (322).

2. The power injector of claim 1, wherein said orientation sensor (306) comprises an accelerometer.

3. The power injector of any one of claims 1-2, wherein said output of said orientation sensor (306) is indicative of a change in orientation of said power injector.

4. The power injector of any one of claims 1-3, wherein said output of said orientation sensor (306) is indicative of a change in orientation of at least one of a powerhead (304) of said power injector and a syringe interconnected with said syringe plunger driver.

5. The power injector of any one of claims 1-4, further comprising a powerhead (304) that comprises said syringe plunger driver (304) and said orientation sensor (306).

6. The power injector of any one of claims 1-5, wherein said first condition, is when said power injector has experienced a predetermined change in orientation.

7. The power injector of any one of claims 1-6, wherein said first condition is when at least one of a powerhead (304) of said power injector and a syringe (306) installed on said powerhead is moving toward a tilted up position.

8. A method of operation for a power injector comprising:
providing a power injector according to claim 1;
monitoring for an occurrence of a first condition, wherein said first condition is when said power injector experiences a predetermined change in orientation; and
initiating an RF syringe communication between said power injector and an RFID tag of a syringe installed on said power injector in response to said monitoring step identifying an occurrence of said first condition.

9. The method of claim 8, wherein said monitoring step comprises using an accelerometer.

10. The method of claim 8, wherein said monitoring step comprises using an orientation sensor.

11. The method of any one of claims 8-10, wherein said monitoring step comprises monitoring an acceleration of said power injector.

12. The method of any one of claims 8-11, further comprising:
moving said power injector into a first orientation where a discharge nozzle of said syringe projects at least generally upwardly, wherein said moving said power injector into a first orientation step provides an occurrence of said first condition; and
executing at least one of first and second operations with said power injector in said first orientation, said first operation comprising purging air from said syringe and said second operation comprising loading fluid into said syringe.

13. The method of claim 12, further comprising:
moving said power injector into a second orientation after said executing step and where said discharge nozzle of said syringe now projects at least generally downwardly.

14. The method of claim 13, wherein said moving said powerhead into a second orientation step provides an occurrence of said first condition, wherein each said initiating step is executed prior to a discharging step.

## Patentansprüche

1. Powerinjektor umfassend:
einen Spritzenkolbenantrieb, der eine motorisierte Antriebsquelle (304) umfasst,
einen Orientierungssensor (306) und
eine Kommunikationseinrichtung (314), die eine RFID-Lese/Schreib-Einrichtung umfasst,
**dadurch gekennzeichnet, dass**
der Injektor ferner eine Spritzenkommunikationslogik (316) umfasst, die mit dem Orientierungssensor wirkverbunden ist und konfiguriert ist zum Initiieren einer Spritzenkommunikation als Antwort auf ein Identifizieren durch die Spritzenkommunikationslogik eines Auftretens einer ersten Bedingung unter Verwendung einer Ausgabe des Orientierungssensors, wobei die Kommunikationseinrichtung mit der Spritzenkommunikationslogik wirkverbunden ist, wobei der erste Zustand vorliegt, wenn der Powerinjektor eine vorbestimmte Orientierungsänderung erfährt, und wobei die Spritzenkommunikation eine Kommunikation zwischen der Kommunikationseinrichtung und einer Spritze (320) ist, die an dem Powerinjektor montiert ist und ein integriertes RFID-Tag (322) aufweist.

2. Powerinjektor nach Anspruch 1, wobei der Orientierungssensor (306) einen Beschleunigungsaufnehmer umfasst.

3. Powerinjektor nach einem der Ansprüche 1-2, wobei die Ausgabe des Orientierungssensors (306) auf eine Änderung der Orientierung des Powerinjektors schließen lässt.

4. Powerinjektor nach einem der Ansprüche 1-3, wobei die Ausgabe des Orientierungssensors (306) auf eine Änderung der Orientierung von zumindest einem von einem Antriebskopf (304) des Powerinjektors und einer mit dem Spritzenkolbenantrieb verbundenen Spritze schließen lässt.

5. Powerinjektor nach einem der Ansprüche 1-4, ferner umfassend einen Antriebskopf (304), der den Spritzenkolbenantrieb (304) und den Orientierungssensor (306) umfasst.

6. Powerinjektor nach einem der Ansprüche 1-5, wobei der erste Zustand vorliegt, wenn der Powerinjektor eine vorbestimmte Orientierungsänderung erfahren hat.

7. Powerinjektor nach einem der Ansprüche 1-6, wobei der erste Zustand vorliegt, wenn zumindest einer von einem Antriebskopf (304) des Powerinjektors und einer an dem Antriebskopf montierten Spritze (306) sich in Richtung einer nach oben gekippten Stellung bewegt.

8. Verfahren zum Betrieb eines Powerinjektors, umfassend:
Bereitstellen eines Powerinjektors gemäß Anspruch 1,
Überwachen des Auftretens eines ersten Zustands, wobei der erste Zustand vorliegt, wenn der Powerinjektor eine vorbestimmte Orientierungsänderung erfährt, und
Initiieren einer RF-Spritzenkommunikation zwischen dem Powerinjektor und einem RFID-Tag einer an dem Powerinjektor montierten Spritze als Antwort auf ein Identifizieren durch den Überwachungsschritt des Auftretens des ersten Zustands.

9. Verfahren nach Anspruch 8, wobei der Überwachungsschritt die Verwendung eines Beschleunigungsaufnehmers umfasst.

10. Verfahren nach Anspruch 8, wobei der Überwachungsschritt die Verwendung eines Orientierungssensors umfasst.

11. Verfahren nach einem der Ansprüche 8-10, wobei der Überwachungsschritt die Überwachung einer Beschleunigung des Powerinjektors umfasst.

12. Verfahren nach einem der Ansprüche 8-11, ferner umfassend:
Bewegen des Powerinjektors in eine erste Orientierung, bei der eine Auslassdüse der Spritze zumindest allgemein nach oben ragt, wobei der Schritt des Bewegens des Powerinjektors in eine erste Orientierung ein Auftreten des ersten Zustands bereitstellt, und
Ausführen von mindestens einem von einem ersten und einem zweiten Vorgang mit dem Powerinjektor in der ersten Ausrichtung, wobei der erste Vorgang das Spülen von Luft aus der Spritze umfasst und der zweite Vorgang das Laden von Fluid in die Spritze umfasst.

13. Verfahren nach Anspruch 12, ferner umfassend:
Bewegen des Powerinjektors in eine zweite Ausrichtung nach dem Ausführungsschritt, wobei die Auslassdüse der Spritze nun zumindest allgemein nach unten ragt.

14. Verfahren nach Anspruch 13, wobei das Bewegen des Antriebskopfs in einem zweiten Orientierungsschritt ein Auftreten des ersten Zustands bereitstellt, bei dem jeder Initiierungsschritt vor einem Auslassschritt ausgeführt wird.

## Revendications

1. Injecteur électrique, comprenant :
un dispositif d'entraînement de piston de seringue comprenant une source d'entraînement motorisée (304) ;
un détecteur d'orientation (306) ; et
un dispositif de communication (314) comprenant un dispositif de lecture /écriture RFID ; **caractérisé en ce que** ledit injecteur comprend en outre une logique de communication avec une seringue (316) interconnectée de manière fonctionnelle audit détecteur d'orientation et configurée pour établir une communication avec une seringue en réponse à ladite logique de communication avec une seringue identifiant une occurrence d'un premier état en utilisant une sortie dudit détecteur d'orientation, dans lequel ledit dispositif de communication est interconnecté de manière fonctionnelle à ladite logique de communication avec une seringue, dans lequel ledit premier état apparaît lorsque ledit injecteur électrique subit un changement prédéterminé d'orientation, et dans lequel ladite communication avec une seringue est une communication entre ledit dispositif de communication et
une seringue (320) qui est montée sur l'injecteur électrique et qui a une étiquette RFID intégrée (322).

2. Injecteur électrique selon la revendication 1, dans lequel ledit détecteur d'orientation (306) comprend un accéléromètre.

3. Injecteur électrique selon l'une quelconque des revendications 1 et 2, dans lequel ladite sortie dudit détecteur d'orientation (306) est représentative d'un changement d'orientation dudit injecteur électrique.

4. Injecteur électrique selon l'une quelconque des revendications 1 à 3, dans lequel ladite sortie dudit détecteur d'orientation (306) est représentative d'un changement d'orientation d'au moins l'un parmi une tête de pompe (304) dudit injecteur électrique et une seringue interconnectée audit dispositif d'entraînement de piston de seringue.

5. Injecteur électrique selon l'une quelconque des revendications 1 à 4, comprenant en outre une tête de pompe (304) qui comprend ledit dispositif d'entraînement de piston de seringue (304) et ledit détecteur d'orientation (306).

6. Injecteur électrique selon l'une quelconque des revendications 1 à 5, dans lequel ledit premier état apparaît quand ledit injecteur électrique a subi un changement prédéterminé d'orientation.

7. Injecteur électrique selon l'une quelconque des revendications 1 à 6, dans lequel ledit premier état apparaît quand au moins l'un des éléments suivants, à savoir une tête de pompe (304) dudit injecteur électrique et une seringue (306) installée sur ladite tête de pompe, se déplace vers une position inclinée vers le haut.

8. Procédé de fonctionnement d'un injecteur électrique comprenant les étapes consistant à :
fournir un injecteur électrique selon la revendication 1 ;
surveiller une occurrence d'un premier état, dans lequel ledit premier état apparaît lorsque ledit injecteur électrique subit un changement prédéterminé d' orientation ; et
établir une communication RF avec une seringue entre ledit injecteur électrique et une étiquette RFID d'une seringue installée sur ledit injecteur électrique en réponse à ladite étape de surveillance identifiant une occurrence dudit premier état.

9. Procédé selon la revendication 8, dans lequel ladite étape de surveillance comprend l'utilisation d'un accéléromètre.

10. Procédé selon la revendication 8, dans lequel ladite étape de surveillance comprend l'utilisation d'un détecteur d'orientation.

11. Procédé selon l'une quelconque des revendications 8 à 10, dans lequel ladite étape de surveillance comprend une surveillance d'une accélération dudit injecteur électrique.

12. Procédé selon l'une quelconque des revendications 8 à 11, comprenant en outre les étapes consistant à :
déplacer ledit injecteur électrique dans une première orientation dans laquelle une buse de décharge de ladite seringue fait saillie au moins généralement vers le haut, dans lequel ledit déplacement dudit injecteur électrique lors d'une première étape d'orientation fournit une occurrence dudit premier état ; et
exécuter au moins l'une des première et deuxième opérations avec ledit injecteur électrique dans ladite première orientation, ladite première opération comprenant une purge de l'air de ladite seringue et ladite deuxième opération comprenant un chargement d'un fluide dans ladite seringue.

13. Procédé selon la revendication 12, comprenant en outre :
le déplacement dudit injecteur électrique dans une deuxième orientation après ladite étape d'exécution et une fois que ladite buse de décharge de ladite seringue fait saillie au moins généralement vers le bas.

14. Procédé selon la revendication 13, dans lequel ledit déplacement de ladite tête de pompe lors d'une deuxième étape d'orientation fournit une occurrence dudit premier état, dans lequel chacune desdites étapes d'établissement de communication est exécutée avant une étape de décharge.
